# EUROPEAN PATENT SPECIFICATION

(11) **EP 1 169 336 B1**
(45) Date of publication and mention of the grant of the patent: **01.02.2006**
(21) Application number: 00925219.8
(22) Date of filing: 17.04.2000
(51) Int. Cl.: C07J 15/00, A61K 31/565, C07J 41/00, C07J 43/00, C07J 53/00, A61P 5/30, A61P 15/02, A61P 19/10, A61P 15/08, A61P 9/10, A61P 9/14, A61P 1/04, A61P 25/28, A61P 29/00, A61P 37/00

(54) **ENT-STEROIDS AS SELECTIVELY ACTIVE ESTROGENS**
ENT-STEROIDE ALS SELEKTIV WIRKSAME ESTROGENE
ENT-STEROIDES UTILISES COMME OESTROGENES SELECTIVEMENT ACTIFS

(30) Priority: 15.04.1999 DE 19917930
(43) Date of publication of application: 09.01.2002
(73) Proprietor: Schering Aktiengesellschaft, 13353 Berlin (DE)
(72) Inventor: SCHWARZ, Siegfried, D-07743 Jena (DE); KOSEMUND, Dirk, D-99091 Erfurt (DE); MÜLLER, Gerd, D-07745 Jena (DE); RICHTER, Margit, D-07745 Jena (DE); PETERS, Olaf, D-07745 Jena (DE); DROESCHER, Peter, D-99425 Weimar (DE); ELGER, Walter, D-14195 Berlin (DE); HILLISCH, Alexander, D-07745 Jena (DE); BÖMER, Ulf, D-13347 Berlin (DE); FRITZEMEIER, Karl-Heinrich, D-13505 Berlin (DE); HEGELE-HARTUNG, Christa, D-45470 Mülheim an der Ruhr (DE)
(86) International application number: PCT/EP2000/003470
(87) International publication number: WO 2000/063228

(56) References cited:
- EP-A- 0 033 561
- EP-A- 0 069 424
- EP-A- 0 675 134
- EP-A- 0 676 202
- DD-A- 235 394
- DE-A- 2 037 155
- FR-A- 1 453 212
- FR-A- 1 509 931
- US-A- 4 330 540
- BUZBY, GEORGE C., JR. ET AL: "Totally synthetic steroid hormones. XIII. The chemical resolution of some racemic estrane, 13.beta.-ethylgonane, and 13.beta.-n-propylgonane derivatives and the preparation of some estrane and 13.beta.-ethylgonane derivatives of unnatural configuration" J. MED. CHEM. (1967), 10(2), 199-204 , XP002143508
- RUFER, CLEMENS ET AL: "Total synthesis of optically active steroids. IV. Total synthesis of natural and enantiomeric 8.alpha.-estrane derivatives and racemic 8.alpha.,14.beta.- and 8.alpha.,9.beta.,14.beta.-estrane (or 13.alpha.-estrane) derivatives" JUSTUS LIEBIGS ANN. CHEM. (1967), 705, 211-26 , XP002143509
- SISENWINE, SAMUEL F. ET AL: "Excretion and stereoselective biotransformations of dl-, d- and l-norgestrel in women" DRUG METAB. DISPOS. (1975), 3(3), 180-8 , XP000933474
- MEYERSON, BENGT J.: "Optical isomers of estrogen and estrogen-inhibitors as tools in the investigation of estrogen action on the brain" STEROID HORM. BRAIN FUNCT., PROC. CONF. (1971), MEETING DATE 1970, 237-45. EDITOR(S): SAWYER, CHARLES H. PUBLISHER: UNIV. CALIF., BERKELEY, CALIF. , XP000933570
- HOFFMANN, SIEGFRIED ET AL: "Estrogen-mesogens - 3-[4-n-alkoxybenzoyloxy]-17-[4-n-alkyl- and -alkoxyphenylimino]-estra-1,3,5(10)-triene s" Z. CHEM. (1986), 26(7), 252-4 , XP000933713
- HUTCHINSON, JOHN H. ET AL: "Enantiospecific synthesis of estrone" CAN. J. CHEM. (1987), 65(1), 1-6 , XP002143513
- HOFFMANN, SIEGFRIED ET AL: "Estrogen mesogens. V. Stilbestrol and enantiomeric estradiol derivatives" Z. CHEM. (1979), 19(2), 62-3 , XP000933469
- ENEV V S ET AL: "The first Lewis acid mediated asymmetric Torgov cyclisation" TETRAHEDRON: ASYMMETRY,NL,ELSEVIER SCIENCE PUBLISHERS, AMSTERDAM, vol. 9, no. 15, 7 August 1998 (1998-08-07), pages 2693-2699, XP004132765 ISSN: 0957-4166
- ASAKO, TSUNEHIKO ET AL: "Improved synthesis of optically active steroids" CHEM. PHARM. BULL. (1973), 21(1), 107-11 , XP000933554
- RUFER, CLEMENS ET AL: "Total synthesis of optically active steroids. II. Total synthesis of natural estradiol methyl ether" JUSTUS LIEBIGS ANN. CHEM. (1967), 701, 206-16, XP002143516
- BONFILS, A. ET AL: "RU 3117 a steroidal compound with high affinity for sigma sites in rat testis membranes" J. STEROID BIOCHEM. MOL. BIOL. (1996), 59(1), 49-54 , XP000933547
- ANSTEAD G M ET AL: "The estradiol pharmacophore: Ligand structure-estrogen receptor binding affinity relationships and a model for the receptor binding site" STEROIDS: STRUCTURE, FUNCTION, AND REGULATION,US,ELSEVIER SCIENCE PUBLISHERS, NEW YORK, NY, vol. 62, no. 3, 1 March 1997 (1997-03-01), pages 268-303, XP004057108 ISSN: 0039-128X

## Description

### Field of the Invention

This invention relates to new compounds as pharmaceutical active ingredients, which have in vitro a higher affinity to estrogen receptor preparations from rat prostates than to estrogen receptor preparations from rat uteri and in vivo preferably a preferential action on bone rather than the uterus and/or pronounced action with respect to stimulation of the expression of 5HT2a-receptors and transporters, their production, their therapeutic use as well as pharmaceutical dispensing forms that contain the new compounds.

The chemical compounds are novel, steroidal, tissue-selective estrogens.

### Background of the Invention

Established estrogen therapies for treatment of hormone-deficiency-induced symptoms and the protective action of estrogens on bones, brains, vessels and other organ systems:

The efficiency of estrogens in the treatment of hormone-deficiency-induced symptoms such as hot flashes, atrophy of estrogen target organs and incontinence, as well as the successful use of estrogen therapies for prevention of bone mass loss in peri- and postmenopausal women, is well documented and generally accepted (Grady, D. et al., 1992, Ann Intern Med 117: 1016-1037). It is also well documented that estrogen replacement therapy in postmenopausal women or in women with ovarian dysfunction that is caused in some other way reduces the risk of cardiovascular diseases compared to non-estrogen-treated women (Grady et al., loc. cit.).

In addition, more recent studies confirm a protective action of estrogens against neurodegenerative diseases, such as, e.g., Alzheimer's disease (Henderson 1997, Neurology 48 (Suppl 7): S27-S35; Birge 1997, Neurology 48 (Suppl 7): S36-S41), a protective action with respect to brain functions, such as memory and learning capacity (McEwen et al. 1997, Neurology 48 (Suppl 7): S8-S15; Sherwin 1997, Neurology 48 (Suppl 7): S21-S26), as well as against hormone-deficiency-induced mood swings (Halbreich 1997, Neurology 48 (Suppl 7): S16-S24).

In addition, estrogen replacement therapy has proven effective relative to the reduction of the incidence of colorectal carcinoma (Calle, E. F. et al., 1995, J Natl Cancer Inst 87: 517-523).

In conventional estrogen or hormone replacement therapy (= HRT), natural estrogens, such as estradiol, and conjugated estrogens that consist of equine urine are used either by themselves or in combination with a gestagen. Instead of the natural estrogens, derivatives that are obtained by esterification, such as, e.g., 17β-estradiol-valerate, can also be used.

Because of the stimulating action of the estrogens that are used on the endometrium, which results in an increase of the risk of endometrial carcinoma (Harlap, S. 1992, Am J Obstet Gynecol 166: 1986-1992), estrogen/gestagen combination preparations are preferably used in hormone replacement therapy. The gestagenic component in the estrogen/gestagen combination avoids hypertrophy of the endometrium, but the occurrence of undesirable intracyclic menstrual bleeding is also linked to the gestagen-containing combination.

Selective estrogens represent a more recent alternative to the estrogen/gestagen combination preparations. Up until now, selective estrogens have been defined as those compounds that have an estrogen-like effect on the brain, bones and vascular system, owing to their antiuterotrophic (i.e., antiestrogenic) partial action, but they do not have a proliferative effect on the endometrium.

A class of substances that partially meet the desired profile of a selective estrogen are the so-called "Selective Estrogen Receptor Modulators" (SERM) (R. F. Kauffman, H. U. Bryant 1995, DNAP 8 (9): 531-539). In this case, these are partial agonists of estrogen receptor subtype "ERα." This substance type is ineffective, however, with respect to the treatment of acute postmenopausal symptoms, such as, e.g., hot flashes. As an example of a SERM, the raloxifene that was recently introduced for the indication of osteoporosis can be mentioned.

### Estrogen Receptor Beta (ERβ)

Estrogen receptor (ERβ) was recently discovered as a second subtype of the estrogen receptor (Kuiper et al. (1996), Proc. Natl. Acad. Sci. 93: 5925-5930; Mosselman, Dijkema (1996) Febs Letters 392: 49-53; Tremblay et al. (1997), Molecular Endocrinology 11: 353-365). The expression pattern of ERβ differs from that of the ERα (Kuiper et al. (1996), Endocrinology 138: 863-870). ERβ thus predominates over ERα in the rat prostate, while ERα predominates over ERβ in the rat uterus. Areas in which in each case only one of the two ER-subtypes is expressed were identified in the brain (Shugrue et al. (1996), Steroids 61: 678-681; Li et al. (1997), Neuroendocrinology 66:63-67). ERβ is expressed in, i.a., areas that are considered to be important for cognitive processes and "mood" (Shugrue et al. 1997, J Comparative Neurology 388: 507-525).

Molecular targets for ERβ in these brain areas could be the 5HT2a-receptor and the serotonin transporter (G. Fink & B. E. H. Sumner 1996 Nature 383:306; B. E. H. Sumner et al. 1999 Molecular Brain Research, in press). The neurotransmitter serotonin (5-hydroxytryptamine) is involved in the regulation of a considerable number of processes, which can be impaired in menopause. In particular, the effects of menopause on emotion and cognition are connected with the serotoninergic system. Estrogen replacement therapy has proven effective with respect to treatment of these estrogen deficiency-produced symptoms, possibly by modulation of serotonin receptor and transporter expression.

Other organ systems with comparatively higher ERβ-expression comprise the bones (Onoe, Y. et al., 1997, Endocrinology 138: 4509-4512), the vascular system (Register, T. C.; Adams, M. R. 1998, J. Steroid Molec Biol 64: 187-191), the urogenital tract (Kuiper, G. J. M. et al. 1997, Endocrinology 138: 863-870), the gastrointestinal tract (Campbell-Thopson 1997, BBRC 240: 478-483), as well as the testis (Mosselmann, S. et al. 1996 Febs Lett 392 49-53) including the spermatides (Shugrue et al. 1998, Steroids 63: 498-504). The tissue distribution suggests that estrogens regulate organ functions via ERβ. The fact that ERβ is functional in this respect also follows by studies in ERα- (ERKO) or ERβ-(βERKO)-knockout mice: ovariectomy produces bone mass loss in ERKO-mice, which can be cancelled out by estrogen substitution (Kimbro et al. 1998, Abstract OR7-4, Endocrine Society Meeting New Orleans). Estradiol in the blood vessels of female ERKO mice also inhibits vascular media and smooth muscle cell proliferation (lafrati, M. D. et al. 1997, Nature Medicine 3: 545-548). These protective actions of estradiol are carried out in the ERKO mouse presumably via ERβ.

Observations of βERKO mice provide an indication on a function of ERβ in the prostate and bladder: in the case of older male mice, symptoms of prostate and bladder hyperplasia occur (Krege, J. H. et al. 1998, Proc Natl Acad Sci 95: 15677-15682). in addition, female ERKO mice (Lubahn, D. B. et al. 1993, Proc Natl Acad Sci 90: 11162-11166) and male ERKO mice (Hess, R. A. et al. 1997, Nature 390: 509-512) as well as female βERKO mice (Krege, J. H., 1998) have fertility disorders. Consequently, the important function of estrogens with respect to maintaining testis and ovary functions as well as fertility is confirmed.

Westerlind et al., 1998, describe a differential action of 16α-hydroxyestrone on the bones, on the one hand, and reproductive organs of female rats, on the other (Westerlind et al. 1998, J Bone and Mineral Res 13: 1023-1031).

Some studies showed that 16α-hydroxyestrone binds three times better to the human estrogen receptor β (ERβ) than to the human estrogen receptor α (ERα). The RBA value of the substance on the rat prostate estrogen receptor is five times better than the RBA value of the substance on the rat uterus estrogen receptor. According to some findings, the dissociation of the substance that is described by Westerlind can be attributed to their preference for ERβ rather than ERα.

It was possible to achieve a selective estrogen action on specific target organs by subtype-specific ligands based on the different tissue or organ distribution of the two subtypes of the ERs. Substances with preference for ERβ compared to ERα in the in vitro receptor binding test were described by Kuiper et al. (Kuiper et al. (1996), Endocrinology 138: 863-870). A selective action of subtype-specific ligands of the estrogen receptor on estrogen-sensitive parameters in vivo was not previously shown.

The object of this invention is therefore to prepare compounds that have in vitro a dissociation with respect to the binding to estrogen receptor preparations from rat prostates and rat uteri and that have in vivo preferably a dissociation with respect to bones rather than the uterus action. The compounds are to have in vitro a higher affinity to estrogen receptor preparations from rat prostates than to estrogen receptor preparations from rat uteri and in vivo preferably a higher potency with respect to protection against hormone-deficiency-induced bone mass loss in comparison to uterus-stimulating action and/or pronounced action with respect to stimulation of the expression of 5HT2a-receptors and transporters.

In the broader sense, a structure-action relationship, which allows for access to compounds that have the above-formulated pharmacological profile of better estrogenic action on bones than on the uterus, is to be made available by this invention.

The invention refers to the following compounds:
ent-Estriol
ent-Estriol-3-sulfamate
ent-Estriol-3-(N-acetyl)sulfamate
ent-Estriol-3,16,17-tripropionate
ent-Estrone-3-sulfamate
ent-Estrone-3-(N-acetyl)sulfamate
ent-Estradiol-3-sulfamate
ent-Estradiol-3,17-disulfamate
ent-Estradiol-3-(N-acetyl)sulfamate
ent-Estradioi-3,17-bis(N-acetyl)sulfamate
ent-Estrone-(N-propionyl)sulfamate
ent-Estradiol-3-(N,N-dimethyl)sulfamate
ent-Estradiol-3-(N,N-diethyl)sulfamate
ent-Estradiol-3-pyrrolidinosulfonate
ent-Estradiol-17-valerianate
ent-Estradiol-17-decanoate
ent-3,17β-Dihydroxy-estra-1,3,5(10)-trien-2-yl-sulfamate
ent-16α-Hydroxy-17-oxo-estra-1,3,5(10)-trien-3-yl-sulfamate
ent-16α-Hydroxy-3-methoxy-estra-1,3,5 (10)-trien-17-one
ent-Estra-1,3,5(10)-triene-3,16α-diol
ent-3-Methoxy-estra-1,3,5(10)-triene-16α,17β-diol
ent-2-Methoxy-estra-1,3,5(10)-triene-3,17β-diol
ent-17β-Hydroxy-14α,15α-methylen-estra-1,3,5(10)-trien-3-yl-(N-acetyl)sulfamate
ent-14α,15α-Methylen-estra-1,3,5(10)-triene-3,17β-diol
ent-14β,15β-Methylen-estra-1,3,5(10)-triene-3,17β-diol
ent-14α,15α-Methylen-estra-1,3,5(10),8-tetraene-3,17α-diol
ent-17α-Hydroxy-14α,15α-methylen-estra-1,3,5(10),8-tetraen-3-yl-(N-acetyl)sulfamate
ent-14β,15β-Methylen-estra-1,3,5(10),8-tetraene-3,17β-diol
ent-16α-Hydroxy-estra-1,3,5(10)-trien-3-yl-sulfamate
ent-16α-Hydroxy-estra-1,3,5(10)-trien-3-yl-benzoate
ent-3-Hydroxy-estra-1,3,5(10)-trien-17α-yl-undecanoate
ent-17β-Hydroxy-estra-1,3,5(10),8-tetraen-3-yl-sulfamate
ent-17β-Hydroxy-estra-1,3,5(10),8-tetraen-3-yl-(N-butyryl)sulfamate
ent-Estra-1,3,5(10),8-tetraene-3,17α-diol
ent-Estra-1,3,5(10),8-tetraene-3,17β-diol
ent-Estra-1,3,5(10),9(11)-tetraene-3,17β-diol
ent-Estra-1,3,5(10),7-tetraene-3,17β-diol
ent-Estra-1,3,5(10),7-tetraene-3,17α-diol
ent-17β-Hydroxy-estra-1,3,5(10),7-tetraen-3-yl-sulfamate
ent-Estra-1,3,5(10),6-tetraene-3,17β-diol
ent-Estra-1,3,5(10),6,8-tetraene-3,17β-diol
ent-Estra-1,3,5(10),6,8-tetraene-3,17α-diol
ent-Estra-1,3,5(10),8,14-pentaene-3,17β-diol-3-butyrate
ent-Estra-1,3,5(10),8,14-pentaene-3,17α-diol
ent-7α-Methylestra-1,3,5(10)-triene-3,17β-diol
ent-9α-Methylestra-1,3,5(10)-triene-3,17β-diol
ent-9α-Ethylestra-1,3,5(10)-triene-3,17β-diol
ent-11β-Fluoroestra-1,3,5(10)-triene-3,17β-diol
ent-Estra-1,3,5(10)-triene-3,16β-diol

The invention also relates to uses of the ent-steroids of general formula I 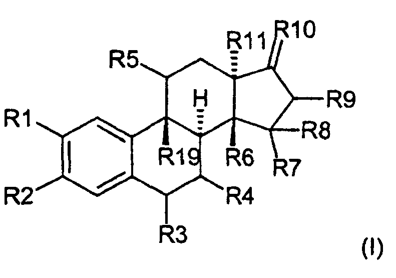
in which
- R¹: means a hydrogen atom;
a group R¹²-O-, whereby R¹² means a hydrogen atom or a hydrocarbon radical with up to 5 carbon atoms, which can contain a C-C-double bond or a C-C-triple bond;
a group R¹³SO₂-O-, in which R¹³ is an R¹⁴R¹⁵N group, whereby R¹⁴ and R¹⁵, independently of one another, mean a hydrogen atom, a C₁-C₅ alkyl radical, a group C(O)R¹⁶, in which R¹⁶ represents a hydrocarbon radical with up to 12 carbon atoms, which can additionally contain up to three double bonds and/or triple bonds, a C₃-C₇ cycloalkyl radical, an aryl radical or a combination thereof or, together with the N-atom mean a polymethylenimino radical with 4 to 6 C atoms or a morpholino radical;
- R²: means a group R¹²-O-, R¹³SO₂-O- or -O-C(O)R¹⁶, with R¹² , R¹³ and R¹⁶ in each case in the meaning that is indicated under R¹;
- R³, R⁴, R⁵, R⁸ and R⁹,: independently of one another, mean a hydrogen atom, a halogen atom, a group R¹²-O-, R¹³SO₂-O-, or - R¹⁶, with R¹² , R¹³ and R¹⁶ in each case in the meaning that is indicated under R¹;
- R⁶: means a β-position hydrogen atom and
- R⁷: means a hydrogen atom
or
- R⁶ and R⁷: together mean an α- or β-methylene group;
- R¹⁰: means two hydrogen atoms; two halogen atoms; a hydrogen atom and a halogen atom; a group =CR¹⁷R¹⁸, in which R¹⁷ and R¹⁸, represent a hydrogen atom and a halogen atom; a hydrogen atom and a group R¹²-O-; a hydrogen atom and a group R¹³SO₂-O-; a group R¹² and a group -O-C(O)R¹⁶; a group R¹² and a hydroxyl group; with R¹², R¹³ and R¹⁶ in each case in the meaning that is indicated under R¹; an oxygen atom;
- R¹¹: means a hydrogen atom, a methyl or ethyl group;
- R¹⁹: means a hydrogen atom or a hydrocarbon radical with up to five carbon atoms, which can be partially or completely fluorinated and which can contain a C-C double bond or a C-C triple bond;
as ER-β selective Estrogens for the manufacture of a medicament for treatment of ERβ related diseases as defined in the following and subject to the disclaimer mentional herein with respect to medicaments for the treatment of male infertility.

A variant of the invention calls for compounds of general formula I that are derived from ent-13-alkylgonane.

The compounds of general formula I and the alkylgonanes with an aromatic A-ring can have one or more (additional) double bonds in rings B, C, D of the steroid skeleton.

The compounds of general formula I can comprise one or more double bonds in positions 6, 7; 7, 8; 8, 9; 9, 11; 11, 12; 8, 14; 14, 15 and 15, 16.

According to a further embodiment of the invention, the ent-13-alkylgonanes with an aromatic A-ring carry a 3-hydroxy group (R²).

In addition, these ent-13-alkylgona-1,3,5(10)-trien-3-ols can have a 17α - or 17β -hydroxyl group, i.e., R¹⁰ stands for a hydrogen atom and a hydroxyl group.

A hydroxyl group can also stand in the ent-13-alkylgona-1,3,5(10)-trien-3-ols in 16-position (R⁹).

The two last-mentioned variants can also be produced simultaneously according to the invention; these are then thus 3,16,17-triols.

In addition, there is the possibility that the ent-1 3-alkylgona-1 ,3,5(10)-trien-3-ols have a 17-keto group; R¹⁰ thus stands for an oxygen atom.

In 13-position (R¹¹), the compounds according to the invention can carry primarily a methyl or ethyl group.

The halogen atom of substituents R³, R⁴. R⁵, R⁸ and R⁹ and in substituent combination R¹⁰ can be a fluorine, chlorine, bromine or iodine atom, preferably a fluorine atom.

Preferred according to this invention is the use of the compounds of general formula I below:
ent-Estriol
ent-Estriol-3-sulfamate
ent-Estriol-3-(N-acetyl)sulfamate
ent-Estriol-3,16,17-tripropionate
ent-Estrone-3-sulfamate
ent-Estrone-3-(N-acetyl)sulfamate
ent-Estradiol-3-sulfamate
ent-Estradiol-3,17-disulfamate
ent-Estradiol-3-(N-acetyl)sulfamate
ent-Estradiol-3,17-bis-(N-acetyl)sulfamate
ent-Estrone-(N-propionyl)sulfamate
ent-Estradiol-3-(N,N-dimethyl)sulfamate
ent-Estradiol-3-(N,N-diethyl)sulfamate
ent-Estradiol-3-pyrrolidinosulfonate
ent-Estradiol-17-valerianate
ent-Estradiol-17-decanoate
ent-3,17β-Dihydroxy-estra-1,3,5(10)-trien-2-yl-sulfamate
ent-16α-Hydroxy-17-oxo-estra-1,3,5(10)-trien-3-yl-sulfamate
ent-16α-Hydroxy-3-methoxy-estra-1,3,5(10)trien-17-one
ent-Estra-1,3,5(10)-triene-3,16α-diol
ent-3-Methoxy-estra-1,3,5(10)-triene-16α,17β-diol
ent-2-Methoxy-estra-1,3,5(10)-triene-3,17β-diol
ent-17β-Hydroxy-14,15-methylen-estra-1,3,5(10)-trien-3-yl-(N-acetyl)sulfamate ent-14α,15α-Methylen-estra-1,3,5(10)-triene-3,17β-diol
ent-14β,15β-Methylen-estra-1,3,5(10)-triene-3,17β-diol
ent-14α,15α-Methylen-estra-1,3,5(10),8-tetraene-3,17α-diol
ent-17α-Hydroxy-14α,15α-methylen-estra-1,3,5(10),8-tetraen-3-yl-(N-acetyl)sulfamate
ent-14β,15β-Methyien-estra-1,3,5(10),8-tetraene-3,17β-diol
ent-16α-Hydroxy-estra-1,3,5(10)-trien-3-yl-sulfamate
ent-16α-Hydroxy-estra-1,3,5(10)-trien-3-yl-benzoate
ent-3-Hydroxy-estra-1,3.5(10)-trien-17α-yl-undecanoate
ent-17β-Hydroxy-estra-1,3,5(10),8-tetraen-3-yl-sulfamate
ent-17β-Hydroxy-estra-1,3,5(10).8-tetraen-3-yl-(N-butyryl)-sulfamate ent-Estra-1,3,5(10),8-tetraene-3,17α-diol
ent-Estra-1,3,5(10),8-tetraene-3.17β-diol
ent-Estra-1,3,5(10),9(11)-tetraene-3,17β-diol
ent-Estra-1,3,5(10),7-tetraene-3,17β-diol
ent-Estra-1,3,5(10),7-tetraene-3,17α-diol
ent-17β-Hydroxy-estra-1,3,5(10),7-tetraen-3-yl-sulfamate ent-Estra-1,3,5(10),6-tetraene-3,17β-diol
ent-Estra-1,3,5(10),6,8-tetraene-3,17β-diol
ent-Estra-1,3,5(10),6,8-tetraene-3,17α-diol
ent-Estra-1,3,5(10),8,14-pentaene-3,17β-diol-3-butyrate
ent-Estra-1,3,5(10),8,14-pentaene-3,17α-diol
ent-7α-Methylestra-1,3,5(10)-triene-3,17β-diol
ent-9α-Methylestra-1,3,5(10)-triene-3,17β-diol
ent-9α-Ethylestra-1,3,5(10ytriene-3,17β-diol
ent-11β-Fluoroestra-1,3,5(10)-triene-3,17β-diol
ent-Estra-1,3,5(10)-triene-3,16β-diol
ent-3,16α-Dihydroxyestra-1,3,5(10)-trien-17-one
ent-Estradiol
ent-Estradiol-3-acetate
ent-Estradiol-17-acetate
ent-Estradiol-diacetate
ent-Estradiol-3-benzoate
ent-17α-Ethinyl-estra-1,3,5(10)-triene-3,17-diol
ent-17α-Ethinyl-estra-1,3,5(10)-triene-3,17-diyl-diacetate
ent-3-Hydroxy-estra-1,3,5(10),7-tetraen-17-one
ent-3-Hydroxy-estra-1,3,5(10),6,8-pentaen-17-one
ent-16β-Bromo-3-methoxy-estra-1,3,5(10)-trien-17-one

### Definition of the substituents in the compounds of formula I:

In terms of this invention, an aryl radical is a phenyl, 1- or 2-napthyl radical; and the phenyl radical is preferred.

Unless expressly indicated otherwise, aryl also always includes a heteroaryl radical. Examples of a heteroaryl radical are 2-, 3- or 4-pyridinyl, 2- or 3-furyl, 2- or 3-thienyl, 2- or 3-pyrrolyl, 2-, 4- or 5-imidazolyl, pyrazinyl, 2-, 4- or 5-pyrimidinyl or 3- or 4-pyridazinyl radicals.

Both the aryl and the heteroaryl radical can be substituted.

As substituents for an aryl or heteroaryl radical, for example, a methyl, ethyl, trifluoromethyl, pentafluoroethyl, trifluoromethylthio, methoxy, ethoxy, nitro, cyano, halogen- (fluorine, chlorine, bromine, iodine), hydroxy-, amino-, mono(C₁₋₈ alkyl) or di(C₁₋₈alkyl)amino, whereby both alkyl groups are identical or different, di(aralkyl)amino, whereby both aralkyl groups are identical or different, can be mentioned.

The hydrocarbon radical with up to 5 carbon atoms is a C₁-C₅ alkyl radical, such as, e.g., a methyl, ethyl, propyl, isopropyl, butyl, isobutyl, tert-butyl, pentyl, isopentyl or neopentyl radical or a C₂C₅ alkenyl or C₂-C₅ alkinyl radical, such as, e.g., an ethinyl, propinyl, pentinyl, vinyl or allyl radical.

As representatives of straight-chain or branched-chain hydrocarbon radicals with 1-12 carbon atoms, for example, methyl, ethyl, propyl, isopropyl, butyl, isobutyl, tert-butyl, pentyl, isopentyl, neopentyl, heptyl, hexyl, and decyl can be mentioned; methyl, ethyl, propyl and isopropyl are preferred for the alkyl or hydrocarbon radical.

The alkyl group or the hydrocarbon radical can be fluorinated partially or completely or substituted by 1-5 halogen atoms, hydroxy groups or C₁-C₄ alkoxy groups. As perfluorinated alkyl groups, for example, trifluoromethyl, pentafluoroethyl and nonafluorobutyl can be mentioned. Representatives of the partially fluorinated alkyl groups are, for example, 2,2,2-trifluoroethyl, 5,5,5,4,4-pentafluoropentyl, 9,9,9,8,8,7,7,6,6-nonafluorohexyl, etc.

If a double bond is present in rings B, C and D of the steroid skeleton, the following variants exist:
a double bond between C atoms 6 and 7 or between C atoms 7 and 8 or between C atoms 8 and 9 or between C atoms 9 and 11 or between C atoms 11 and 12 or between C atoms 8 and 14 or between C atoms 14 and 15 or between C atoms 15 and 16; preferably between C atoms 7, 8; 8, 9; 15, 16; and if several double bonds are present in rings B, C and D of the steroid skeleton, the following variants preferably exist: double bonds between C atoms 6 and 7 and C atoms 8 and 9 or between C atoms 8 and 9 and C atoms 14 and 15 or between C atoms 6 and 7, C atoms 8 and 9 as well as C atoms 14 and 15.

The esters of the ent-steroids, used or claimed per se according to the invention have advantages as prodrugs compared to unesterified active ingredients with respect to their method of administration, their type of action, their strength and duration of action.

The ent-steroid-sulfamates according to the invention also have pharmacokinetic and pharmacodynamic advantages. In this connection, effects were already described in the case of sulfamates that are derived from estrogens with natural absolute configurations (J. Steroid Biochem. Molec. Biol., 55, 395-403 (1995); Exp. Opinion Invest. Drugs 7, 575-589 (1998)).

In this patent application, steroids on which the (8α-H, 9β-H, 10α-H, 13α-H, 14β-H)-gonane skeleton is based are described as selective estrogens for treatment of estrogen receptor β-mediated diseases and conditions, and these estrogens have in vitro dissociation with respect to binding to estrogen receptor preparations of rat prostates and rat uteri and have in vivo preferably a dissociation with respect to bone action rather than uterus action: the substances act in a bone-protective manner over a wide dose range without stimulating the uterus. In the same dose range, their liver action is small. In addition, the substances exert estrogen-like action on the vascular system and brain functions.

The compounds according to the invention represent chiral steroids, whose molecular ring skeleton is arranged as a mirror image to naturally occurring steroids. Pairs of optically active compounds, which are identical with respect to their chemical structure, but are distinguished in all chirality centers by a mirror-image arrangement, are named enantiomers. Naturally occurring steroids, or steroid derivatives that are obtained synthetically that have the stereochemistry of the ring skeleton of naturally occurring steroids, do not require any special stereochemical designation with respect to the ring skeleton. For example, the stereochemistry of the estrane skeleton is implicitly contained in the name estrone. The name ent-(enantio-)estrone means that all chirality centers of estrone (C-8, C-9, C-13 and C-14) are arranged inversely. A mixture of the same parts of the respective enantiomers is a racemate.

It is known of ent-steroids that they have little or no hormonal actions.

It was reported of enantiomers of naturally occurring female sex hormone estradiol (ent-estradiol) and the strongly estrogenically active 8α -estradiol (ent-8α-estradiol) that they have antilipemic activity in the case of no feminization.

17α-Ethinylestradiol, which is an extremely strongly active estrogen, shows in its enantio form less than 5% estrogeneity, relative to estrone (J. Med. Chem. 10, 199-204 1967). Furthermore, results of biological testing are given for some other steroids of uninatural configuration in relation to classical estrogenic activity and antilifilm properties.

In the Clauberg Test, ent-17α-chloroethinyl-17β-hydroxy-18a-homo-estr-4-en-3-one proved to be ineffective (Endocrinology 63, 464 (1958), while the compound with naturally absolute configuration is a gestagen.

RU 486 (11β-[4-(dimethylamino)phenyl]-17β-hydroxy-17α-(1-propinyl)estra-4,9-dien-3-one), a steroid with a natural absolute configuration, is distinguished by strong antihormonal actions, ent-RU 486 showed neither antigestagenic nor antiglucocorticoidal activity (Steroids **44**. 519 530 (1984).

ent-Steroids can have actions other than hormonal actions, however.

RU 1868, an ent-estra-1,3,5(10)-triene derivative, was described as a compound that has selective affinity for (+) PPP-binding sites in the membrane of the rat test, but no binding to standard steroid receptors forms (J. Steroid Biochem. Mol. Biol. 59, 49-54 (1996)).

It was reported of ent-steroids that they affect calcium influx in human sperm and thus are potentially suitable for male birth control (EP-A 67 62 02: EP-A 67 51 34). This has anothing to do with the ER-β selective effect as described in the present application.

ent-Steroids showed anti-arrhythmic activities in rats, without the cholesterol level of the plasma being lowered (Steroids 40, 615-623 (1982); US Pat. 4 330 540), neuromuscular inhibition in cats (Eur. J. Med. Chem. 19, 43 47 (1984)), activation of lordosis in ovariectomized rats (Symposium on Steroid Horm. Brain Funct. 1970, Conference Proceedings 237-245 (197)) as well as cholesterol-lowering activity in the absence of any estrogeneity (French Patent 1 45 32 12; Supplement to French Patent 1 33 83 08).

It has been found that the ent-steroids according to the invention are suitable as selective estrogens for treating different conditions and diseases, which are characterized by a higher content of estrogen receptor β than estrogen receptor α in the corresponding target tissue or organ.

The invention also relates to pharmaceutical preparations that contain at least one compound of claim 1 (or physiologically compatible addition salts with organic and inorganic acids of them) and the use of these compounds for the production of pharmaceutical agents, especially for the indications below.

The compounds can be used for the following indications both after oral and parenteral administration.

The novel selective estrogens that are described in this patent can be used as individual components in pharmaceutical preparations or in combination especially with antiestrogens or gestagens. Especially preferred is the combination of selective estrogens with ERα -selective antiestrogens, or with antiestrogens that are peripherally-selectively active, i.e., that do not pass through the blood-brain barriers.

The substances and the pharmaceutical agents that contain them are especially suitable for the treatment of peri- and postmenopausal symptoms, especially hot flashes, sleep disturbances, irritability, mood swings, incontinence, vaginal atrophy, and hormone-deficiency-induced emotional diseases. The substances for hormone substitution and therapy of hormone-deficiency-induced symptoms in the case of surgical, medicinal or ovarian dysfunction that is caused in some other way are also suitable. Prevention of bone mass loss in postmenopausal women, in women who have undergone hysterectomies or in women who were treated with LHRH agonists or LHRH antagonists is also part of this.

The compounds are also suitable for alleviating symptoms of male menopause and female menopause, i.e., for male and female hormone replacement therapy (HRT), specifically both for prevention and for treatment, in addition for treatment of symptoms that are accompanied by a dysmenorrhea as well as for treatment of acne.

In addition, the substances can be used for prophylaxis against hormone-deficiency-induced bone mass loss and osteoporosis, for prevention of cardiovascular diseases, especially vascular diseases such as arteriosclerosis, for inhibition of the proliferation of arterial smooth muscle cells, for treatment of primary pulmonary high blood pressure and for prevention of hormone-deficiency-induced neurodegenerative diseases, such as Alzheimer's disease, as well as hormone-deficiency-induced impairment of memory and learning capacity.

In addition, the compounds can be used for the treatment of male fertility disorders and prostatic diseases whereby due to the incidental disclosure of EP-A-676,202, the present invention excludes the use of the following compounds in the manufacture of a medicament for the treatment of male infertility: (8α,9β,13α,14β)-3-[2-(dimethylamino)-ethoxy]-estra-1,3,5(10)-trien-17-one; (8α,9β,13α,14β)-3-[2-(diethylamino)-ethoxy]-estra-1,3,5(10)-trien-17-one; (8α,9β,13α,14β)-3-[2-(dimethylamino)-ethoxy]-estra-1,3,5(10)-triew(8α,9β,13α,14β)-3-[2-(diethylamino)-ethoxy]-estra-1,3,5(10)-trien and its chlorohydrate; (8α,9β,13α,14β,17α)-3-[2-(diethylamino)-ethoxy]-estra-1,3,5(1.0)-trien-17-ol.

The compounds can also be used in combination with the natural vitamin D3 or with calcitriol analogues for bone formation or as supporting therapies to therapies that cause bone mass loss (for example, therapy with glucocorticoids, chemotherapy).

Finally, the compounds of general formula I can be used in connection with progesterone receptor antagonists, specifically especially for use in hormone replacement therapy and for treatment of gynecological disorders.

A therapeutic product that contains an estrogen and a pure antiestrogen for simultaneous, sequential or separate use for the selective estrogen therapy of perimenopausal or postmenopausal conditions is already described in EP-A 0 346 014.

The amount of a compound of general formula I that is to be administered varies within a wide range and can cover any effective amount. On the basis of the condition that is to be treated and the type of administration, the amount of the compound that is administered can be 0.01 1 µg/kg - 10 mg/kg of body weight, preferably 0.04 µg/kg - 1 mg/kg of body weight, per day.

In humans, this corresponds to a dose of 0.8 µg to 800 mg, preferably 3.2 µg to 80 mg, daily.

According to the invention, a dosage unit contains 1.6 µg to 200 mg of one or more compounds of general formula I.

The compounds according to the invention and the acid addition salts are suitable for the production of pharmaceutical compositions and preparations. The pharmaceutical compositions or pharmaceutical agents contain as active ingredient one or more of the compounds according to the invention or their acid addition salts, optionally mixed with other pharmacologically or pharmaceutically active substances. The production of the pharmaceutical agents is carried out in a known way, whereby the known and commonly used pharmaceutical adjuvants as well as other commonly used vehicles and diluents can be used.

As such vehicles and adjuvants, for example, those are suitable that are recommended or indicated in the following bibliographic references as adjuvants for pharmaceutics, cosmetics and related felds: Ullmans Encyklopcidie der technischen Chemie [Ullman's Encyclopedia of Technical Chemistry], Volume 4 (1953), pages 1 to 39; Journal of Pharmaceutical Sciences, Volume 52 (1963), page 918 ff., issued by Czetsch-Lindenwald, Hilfsstoffe für Pharmazie und angrenzende Gebiete [Adjuvants for Pharmaceutics and Related Fields]; Pharm. Ind., Issue 2, 1961, p. 72 and ff.: Dr. H. P. Fiedler, Lexikon der Hilfsstoffe für Pharmazie, Kosmetik und angrenzende Gebiete [Dictionary of Adjuvants for Pharmaceutics, Cosmetics and Related Fields], Cantor KG, Aulendorf in Württemberg 1971.

The compounds can be administered orally or parenterally, for example intraperitoneally, intramuscularly, subcutaneously or percutaneously. The compounds can also be implanted in the tissue.

For oral administration, capsules, pills, tablets, coated tablets, etc., are suitable. In addition to the active ingredient, the dosage units can contain a pharmaceutically compatible vehicle, such as, for example, starch, sugar, sorbitol, gelatin, lubricant, silicic acid, talc, etc.

For parenteral administration, the active ingredients can be dissolved or suspended in a physiologically compatible diluent. As diluents, very often oils with or without the addition of a solubilizer, a surfactant, a suspending agent or an emulsifying agent are used. Examples of oils that are used are olive oil, peanut oil, cottonseed oil, soybean oil, castor oil and sesame oil.

The compounds can also be used in the form of a depot injection or an implant preparation, which can be formulated so that a delayed release of active ingredient is made possible.

As inert materials, implants can contain, for example, biodegradable polymers, or synthetic silicones such as, for example, silicone rubber. In addition, for percutaneous administration, the active ingredients can be added to, for example, a patch.

For the production of intravaginal systems (e.g., vaginal rings) or intrauterine systems (e.g., pessaries, coils, IUDs, Mirena^{(R)}) that are loaded with active compounds of general formula I for local administration, various polymers are suitable, such as, for example, silicone polymers, ethylene vinyl acetate, polyethylene or polypropylene.

To achieve better bio-availability of the active ingredient, the compounds can also be formulated as cyclodextrin clathrates. For this purpose, the compounds are reacted with α-, β-, or γ-cyclodextrin or derivatives of the latter (PCT/EP95/02656).

According to the invention, the compounds of general formula I can also be encapsulated with liposomes.

### Methods

### Estrogen Receptor Binding Studies

The binding affinity of the new selective estrogens was tested in competitive experiments with use of 3H-estradiol as a ligand to estrogen receptor preparations of rat prostates and rat uteri. The preparation of prostate cytosol and the estrogen receptor test with prostate cytosol was carried out as described by Testas et al. (1981) (Testas, J. et al., 1981, Endocrinology 109: 1287-1289).

The preparation of rat uterus cytosol, as well as the receptor test with the ER-containing cytosol were basically performed as described by Stack and Gorski, 1985 (Stack, Gorski 1985, Endocrinology 117, 2024-2032) with some modifications as described in Fuhrmann et al. (1995) (Fuhrmann, U. et al. 1995, Contraception 51: 45-52).

The substances that are described herein have higher binding affinity to the estrogen receptor of rat prostates than to estrogen receptors of rat uteri. In this case, it is assumed that ERβ predominates in the rat prostates over ERα, and ERα predominates in rat uteri over ERβ. Table 1 shows that the ratio of the binding to prostate and uterus receptor qualitatively coincides with the quotient of relative binding affinity (RBA) to human ER β and ERα of rats (according to Kuiper et al. (1996), Endocrinology 138: 863-870) (Table 1).

In addition, the predictability of the 'prostate-ER versus the uterus-ER test system' was confirmed with respect to tissue-selective action by in vivo studies. Substances with a preference for prostate-ER are dissociated in vivo with respect to bone and uterus action in favor of action on bones (Table 2).

### Bone Studies

Three-month-old female rats are ovariectomized and treated once daily with the test compound immediately after the operation for 28 days. The administration is carried out subcutaneously in arachis oil/ethanol. The animals are sacrificed on the day after the last administration, and tibia as well as uteri are removed. The uteri are weighed, fixed and worked up for histological studies. The determination of bone density is carried out ex vivo on prepared long bones by means of QCT (quantitative computer tomography). The measurements are made at a distance of 4-6 mm from the ball of the joint of the proximal tibia.

The ovariectomy reduces the density of the trabecular bone in the measured area by about 400 mg of Ca²⁺/cm³ to about 300 mg of Ca²⁺/cm³. By treatment with a compound of general formula I according to this invention, the degradation of the bone density is prevented or inhibited. The bone density in the proximal tibia was measured.

Table 2 shows the results for the compound ent-17-estradiol that is to be used according to the invention. It shows a higher binding affinity to the estrogen receptor of rat prostates [ER(RBA) = 6.1] than in the estrogen receptor of rat uteri [ER(RBA) = 0.8]. ent-17-Estradiol reflects this in vivo in the greatly different amounts, which produce a 50% bone protection [30 µg/animal] or a 15% uterus stimulation [300 µg/animal], relative to the bone mass loss, which can be measured in ovariectomized, untreated female rats 28 days after the ovariectomy unlike in intact animals that are subjected to sham operations.

The vascular action of the estrogens according to the invention is determined in the model of the ApoE-knockout mouse, as described by R. Elhage et al., 1997, (Elhage, R. et al. 1997, Arteriosclerosis, Thrombosis and Vascular Biology 17: 2679-2684).

To detect the action of estrogens on the brain function, the oxytocin receptor mRNA expression is used as a surrogate parameter (Hrabovszky, E. et al. 1998, Endocrinology 1339: 2600-2604). Ovariectomized rats are treated for 7 days with the test substance or vehicle (administration: subcutaneous or oral, six times daily). On day 7 after the first administration, the animals are decapitated, the uterus weight is determined, and the oxytocin receptor mRNA level is studied by means of in situ hybridization in suitable brain sections. The ED₅₀ values are determined with respect to stimulation of uterus growth and induction of the oxytocin receptor mRNA.

Another possibility to demonstrate in vivo the dissociated estrogen action of the substances according to the invention consists in the fact that after a one-time administration of the substances in rats, effects on the expression of 5HT2a-receptor and serotonin transporter proteins and mRNA levels in ERβ-rich brain areas can be measured. Compared to the effect on the serotonin receptor and transporter expression, the effect on the LH-secretion is measured. Substances with higher binding to the rat prostate - compared to the rat uterus estrogen receptor - are potent with respect to increasing the expression of serotonin receptors and transporters, in comparison to their positive effect on the LH release. The density of serotonin receptors and transporters is determined in brain sections using radioactive ligands, and the corresponding mRNA is determined using in situ hybridization. The method is described in the literature: G. Fink & B. E. H. Sumner 1996 Nature 383: 306; B. E. H. Sumner et al. 1999 Molecular Brain Research.

### Production of the Compounds According to the Invention

From the commercial standpoint, the bases of the ent-steroids according to the invention are accessible only through total synthesis. In the case of total syntheses, which result in racemates, the bases can be obtained by racemate cleavage with the aid of a chiral adjuvant (e.g., Brit. Pat. 1 139 019; Brit. Pat. 1 159 649; French Pat. 1 526 031: J. Med. Chem. 10, 199-204 (1967). Or they can be obtained by asymmetric synthesis, by prochiral initial products being converted microbiologically into chiral intermediate products and further processed (e.g., Liebigs, Ann. Chem. 701, 206-216 (1967). Another production method consists in the fact that the starting material is a chiral natural product, and the steroid skeleton is built up from the latter (e.g., Can. J. Chem. 65, 1-6, (1987)).

Since the chemistry of ent-steroids is identical to the naturally configured steroids, if no chiral reagents are used, the ent-steroids according to the invention are available from the chemistry of naturally configured steroids according to methods that are known in the art. ent-Estrone or ent-estradiol is thus obtained analogously to the natural hormones from ent-estradiol-3-methyl ether that is produced totally synthetically by oxidation, ether cleavage and reduction (J. Med. Chem. 10, 199-202 (1967)).

ent-Estriol is synthesized from ent-estrone in a way that is similar to the process for the production of estriol (J. Am. Chem. Soc. 76, 2943 (1954)) from ent-estrone.

Analogously, steroids on which the enantio-gonane skeleton is based, and which have additional double bonds in the B-, C- and/or D-ring, are obtained from the corresponding unsaturated enantiomer steroid-intermediate products.

The introduction of variable substituents in rings B, C and D of the enantiomer gonane skeleton is carried out according to the same chemical teaching with which the corresponding gonane derivatives are produced (see, i.a.: Steroide [Steroids], L. F. Fieser, M. Fieser, Verlag Chemie, Weinheim/Bergstr., 1961; Organic Reactions in Steroid Chemistry, J. Fried, J. A. Edwards, Van Nostrand Reinhold Company, New York, Cincinnati, Toronto, London, Melbourne, 1972; Medicinal Chemistry of Steroids, F. J. Zeelen, Elsevier, Amsterdam, Oxford, New York, Tokyo, 1990). This relates to, for example, the introduction of substituents, such as hydroxyl or alkyloxy groups, alkyl, alkenyl or alkinyl groups or fluorine in positions 2, 6, 7, 11, 14, 15, 16 or 17.

The ent-steroid-carboxylic acid esters according to the invention are produced analogously to the esters that are derived from natural steroid active ingredients (see, e.g., Pharmazeutische Wirkstoffe, Synthesen, Patente, Anwendungen [Pharmaceutical Active Ingredients, Syntheses, Patents, Applications]; A. Kleemann, J. Engel', Georg Thieme Verlag Stuttgart 1978. Arzneimittel, Fortschritte [Pharmaceutical Agents, Advances] 1972 to 1985; A. Kleemann, E. Lindner, J. Engel (Editors), VCH 1987, pp. 773-814).

The ent-steroid-sulfamates according to the invention are available in a way that is known in the art from the corresponding ent-steroids by esterification with sulfamoyl chlorides in the presence of a base (Z. Chem. 15, 270-272 (1975); Steroids 61. 710-717 (1996)). Subsequent acylation of the sulfamide group results in the ent-steroidal (N-acyl)sulfamates according to the invention, for which pharmacokinetics advantages have already been demonstrated in the naturally configured series (cf. DE 195 40 233 A1).

The regioselective esterification of polyhydroxylated steroids with N-substituted and N-unsubstituted sulfamoyl chlorides is carried out after partial protection of the hydroxyl groups that are to remain unesterified. Silyl ethers have turned out to be protective groups with suitable selective reactivity in this respect, since silyl ethers are stable under the conditions of sulfamate formation, and the sulfamate group remains intact when the silyl ethers are cleaved again to regenerate the remaining hydroxyl groups that are contained in the molecule (Steroids **61**, 710-717 (1996)).

The production of the sulfamates according to the invention with one or more additional hydroxyl groups in the molecule is also possible in that the starting material is suitable hydroxy-steroid ketones. First, depending on the goal, one or more existing hydroxyl groups are subjected to sulfamoylation. Then, the sulfamate groups can optionally be converted with a desired acyl chloride in the presence of a base into particular (N-acyl)sulfamates. The now present oxosulfamates or oxo-(N-acyl)sulfamates are then converted by reduction into the corresponding hydroxysulfamates or hydroxy-(N-acyl)sulfamates (Steroids **61**, 710-717 (1996)).

Sodium borohydride and borane-dimethyl sulfide complexes have proven to be suitable reducing agents. An example of this procedure is the production of ent-estradiol-3-(N-acetyl or propionyl)sulfamate, as described in Examples 16, 26 and 27.

The compounds of general formula I according to the invention are produced as described in the examples. By an analogous procedure using reagents that are homologous to the reagents that are described in the examples, additional compounds of general formula I can be obtained.

Etherification and/or esterification of free hydroxy groups is carried out according to methods that are common to one skilled in the art.

### Examples

### Example 1

### ent-Estriol

Sodium borohydride (0.508 g) was added within 5 minutes while being stirred to a solution of ent-3,16α -dihydroxy-estra-1,3,5(10)trien-17-one (EP 33 561 A1) (0.508 g) in methanol (50 mL) that was cooled to 0°C. The mixture was allowed to stir for 1 more hour at 0°C, then acetic acid was carefully added drop by drop until a pH of 6 was reached. Then, the solution was completely distilled in a vacuum rotary evaporator (VRV). The residue was extracted with water, dried and recrystallized from ethyl acetate, whereby ent-estriol with a melting point of 275-280°C was obtained.

### Example 2

### ent-Estra 1,3,5(10)-triene-3,16α-diol

N-bromosuccinimide (0.815 g) was added in portions to a solution of ent-3-methoxy-1,3,5(10),16-estratetraene (1.0 g) in dimethyl sulfoxide (38 mL) and water (3.5 mL) that was stirred to +8°C. After 30 minutes, it was diluted with water and extracted with ether. The combined extracts were washed with water, dried on anhydrous sodium sulfate and concentrated by evaporation in a VRV. The residue (ent-17α-bromo-3-methoxy-1,3,5(10)-estra-1,3,5(10)-trien-160-ol) was dissolved in ethanol (29 mL). Hydrazine hydrate (80%, 2.6 mL) and Raney nickel were added to the solution. The mixture was reflux-boiled for 6 hours. Then, Raney nickel was filtered out, and the filtrate was evaporated to the dry state in a VRV. The residue was extracted several times with boiling benzene. The combined extracts were concentrated by evaporation in a VRV. By recrystallization of the residue from n-hexane, ent-3-methoxy-estra-1,3,5(10)-trien-16β-ol was obtained. A solution of diethyl-azodicarboxylate (1.12 mL) and anhydrous benzene (3.5 mL) was added at room temperature drop by drop to a stirred mixture of benzene (14 mL), acetic acid (0.4 mL), triphenylphosphane (1.83 g) and ent-3-methoxy-estra-1,3,5(10)-trien-16β-ol (1.0 g). After 1 hour, precipitate was filtered out, and the filtrate was concentrated by evaporation in a VRV. The residue was chromatographed on silica gel (eluant: benzene), whereby ent-3-methoxy-estra-1,3,5(10)-trien-16α-yl-acetate was obtained, whose hydrolysis with potassium hydroxide (0.209 g) in methanol (7 mL) yielded ent-3-methoxy-estra-1,3,5(10)-trien-16α -ol. Reductive ether cleavage with diisobutylaluminum hydride (3.9 mL) in boiling toluene (7.8 mL) resulted in the title compound, melting point 222-226°C (from methanol).

### ent-Estra-1,3,5(10)-triene-3,17-diols with Additional Double Bonds

### Example 3

### ent-3-Methoxy-estra-1,3,5(10),8,14-pentaen-17α-ol

14α-Hydroxy-3-methoxy-8,14-seco-estra-1,3,5(10),9(11)-tetraen-17-one (50 g) was dissolved at boiling heat in methanol (200 mL). After a clear solution was obtained, concentrated hydrochloric acid (5 mL) was added in drops and heated to boiling for another 3 hours. Then, the product was precipitated with water. Melting point 75-77°C.

### Example 4

### ent-3-Methoxy-1,3,5(10),8-tetraen-17α-ol

ent-3-Methoxy-estra-1,3,5(10),8,14-pentaen-17 -ol (10 g) was dissolved in a tetrahydrofuran/methanol mixture (70:30, v/v, 75 mL). While being exposed to argon gassing, Raney nickel was added to it (water-moistened, 2.20 g). Then, it was hydrogenated with hydrogen with zero pressure. Then, catalyst was filtered out, and the filtrate-was concentrated by evaporation in a VRV to 55 mL, whereby the product crystallized out; melting point 105-115°C.

### Example 5

### ent-Estra-1,3,5(10),9(11)-tetraene-3,17β-diol

Water (200 mL), sodium bicarbonate (64 g), acetone (177 mL) and tetrabutylammonium hydrogen sulfate (0.1 g) were added to a solution of ent-estradiol diacetate (10 g) in dichloromethane (200 mL). The suspension was cooled to +12°C, and then oxone (133 g) was added in portions within 2.5 hours, whereby it was stirred intensively. The stirring continued for 3.5 more hours at +15°C to +20°C. Then, the mixture was filtered, and the separated organic phase was concentrated by evaporation in a VRV. Flash chromatography on silica gel with toluene/ethyl acetate 10:1 (v/v) and crystallization from methanol yielded ent-9-hydroxy-estra-1,3,5(10)-triene-3,17β-diyl-diacetate. ent-9-Hydroxy compound (10 g) was dissolved in dichloromethane (200 mL). The solution was cooled to -20°C and stirred intensively for 2 hours with aqueous sulfuric acid (70%, 1 mL). Then, saturated aqueous sodium bicarbonate solution was added until neutrality was achieved, the organic phase was separated, dried with anhydrous sodium sulfate and concentrated by evaporation in a VRV. The residue was treated with potassium hydroxide (6.65 g) in methanolic solution (133 mL) for 4 hours at 40°C. Then, the bulk of the methanol was distilled off in a VRV, and the title compound was precipitated with water. The purification was carried out by recrystallization from methanol; melting point 186-191°C.

### Example 6

### ent-Estra-1,3,5(10),6-tetraene-3,17-diol

3,5-Dimethylpyrazole (107.9 g) was added in portions to a stirred suspension of chromium-VI-oxide (112.4 g) in dichloromethane (845 mL) that was cooled to -20°C. After another 15 minutes of stirring, a solution of ent-17α-estradiol diacetate (20 g) in dichloromethane (150 mL) was added. It was allowed to react for another 4.5 hours at - 20°C while being stirred. Then, the reaction was completed by adding aqueous sodium hydroxide solution (5N, 460 mL). The phases were separated from one another, the aqueous phase was exhaustively extracted with dichloromethane, the organic solutions were combined, washed in succession with water, dilute hydrochloric acid, saturated aqueous sodium chloride solution and water, dried with anhydrous sodium sulfate and concentrated by evaporation in a VRV to a very large extent. The residue was crystallized with acetone. The substance that was obtained was then further chromatographically purified on a silica gel column with toluene/ethyl acetate/dichloromethane 6:3:1 (v/v/v), whereby ent-6-oxo-estra-1,3,5(10)-triene-3,17α-diyl-diacetate was obtained. This compound (4 g) was subjected at 60°C to hydrolysis with potassium carbonate (11.2 g) in a mixture of methanol (160 mL) and water (28 mL). After working-up, ent-3,17α-dihydroxy-estra-1,3,5(10)-trien-6-one was obtained. By reduction of this compound (2 g) with sodium borohydride (1.3 g) in a mixture of methanol (25 mL) and tetrahydrofuran (20 mL), ent-estra-1,3,5(10)-3, 6α, 17α-triol was obtained. The triol (1.03 g) was dissolved in dimethyl sulfoxide (6 mL), and the solution was heated for 3 hours to 180°C. After cooling to room temperature, it was poured onto ice. The precipitate produced, which was from the title compound, was filtered off, washed with water and dried; melting point 222°C.

### Ether Cleavage of ent-3-Methoxyestratetraene- and 3-methoxy-estrapentaene Steroids

A solution that consists of ent-3-methoxy-estratetraen-17-ol or ent-3-methoxyestrapentaen-17-ol (in each case 1 g), toluene (10 mL) and diisobutylaluminum hydride (5 mL) was reflux-boiled for 3 hours. Then, the reaction solution was cooled to -5°C, and excess diisobutylaluminum hydride was decomposed with ethanol. After subsequent careful addition of water and dilute aqueous hydrochloric acid, the organic phase was separated, washed neutral, dried and evaporated to the dry state in a VRV. Crystallization, optionally after prior flash chromatography, yielded the following compounds according to the invention by way of example:

### Examples 7-14

ent-Estra-1,3,5(10),8-tetraene-3,17β-diol, melting point 130-2°C;
ent-Estra-1,3,5(10),8-tetraene-3,17α-diol, amorphous;
ent-Estra-1,3,5(10),7-tetraene-3,17β-diol, melting point 170-176°C;
ent-Estra-1,3,5(10),7-tetraene-3,17α-diol, melting point 200°C;
ent-Estra-1,3,5(10),6,8-pentaene-3,17β-diol, melting point 241°C;
ent-Estra-1,3,5(10),6,8-pentaene-3,17α-diol, melting point 212-215°C;
ent-Estra-1,3,5(10),8,14-pentaene-3,17β-diol, oil.

### ent-Gona-1,3,5(10)-trien-3-yl-sulfamate and -(N-methyl)sulfamate

Sulfamoyl chloride or N-methylsulfamoyl chloride (in each case 6 mmol) was added drop by drop to a solution of gona-1,3,5(10)-trien-3-ol (1 mmol) and 2,6-di-tert-butyl-pyridine (3 mmol) in dichloromethane (16 mL) while being stirred. After 30 minutes, the organic phase was washed neutral with water, dried on anhydrous sodium sulfate and evaporated to the dry state in a VRV. The crude product was purified by means of flash chromatography. Additional, optionally necessary reaction steps, such as cleavage of protective groups or reduction of a 17-oxo group, followed. Subsequent flash chromatography and recrystallization from acetone/n-hexane yielded the following ent-sulfamates:

### Examples 15-23

ent-Estriol-3-sulfamate, melting point 207-209°C;
ent-Estrone-sulfamate, melting point 200-201°C;
ent-Estradiol-3-sulfamate, melting point 202-203°C;
ent-16α-Hydroxy-estra-1,3,5(10)-trien-3-yl-sulfamate, amorphous;
ent-16α-Hydroxy-17-oxo-estra-1,3,5(10)-trien-3-yl-sulfamate, melting point 180-182°C;
ent-17β-Hydroxy-estra-1,3,5(10),8-tetraen-3-yl-sulfamate, amorphous;
ent-17β-Hydroxy-estra-1,3,5(10),7-tetraen-3-yl-sulfamate, melting point 199-201 °C;
ent-17-Oxo-estra-1,3,5(10)-trien-3-yl-(N-methyl)sulfamate, melting point 190-192°C;
ent-17-Oxo-18α-homo-estra-1,3,5(10)-trien-3-yl-suifamate; melting point 174-176°C.

### ent-Gona-1,3,5(10)-trien-3-yl-(N,N-dialkyl)sulfamates

Aqueous sodium hydroxide solution (40%, 6 mL) is added in drops within 30 minutes to a suspension of ent-estradiol (1 g), dichloromethane (30 mL), N,N-dialkylsulfamoyl chloride (10 molar equivalents), water (3 mL) and triethylbenzyiammonium chloride (0.24 g) while being stirred intensively. It was stirred for another 2 hours, then the organic phase was separated, and it was washed in succession with dilute aqueous hydrochloric acid, saturated aqueous sodium bicarbonate solution and water. After the solution was dried with anhydrous sodium sulfate and concentration by evaporation in a VRV, the residue was recrystallized:

### Examples 24 and 25

ent-Estradiol-3-(N,N-dimethyl)sulfamate, melting point 204-208°C;
ent-Estradiol-3-(N,N-diethyl)sulfamate: melting point 174-177°C.

### Example 26

### ent-Estradiol-3-(N-acetyl)sulfamate

ent-Estrone-sulfamate (2.0 g) was dissolved in pyridine (100 mL). Acetic anhydride (100 mL) was added to the solution, and the mixture was stirred for 2 hours at +23°C. Then, it was decomposed with ice, the precipitate was filtered off, washed neutral with water and dried in a stream of air. Recrystalliza-tion from acetone yielded ent-estrone-(N-acetyl)sulfamate; melting point 219-222°C. The ent-estrone derivative (1g) was reduced at 0°C in a mixture of tetrahydrofuran (100 mL) and methanol (100 mL) with sodium borohydride (0.68 g). After neutralization of the reaction solution with acetic acid (2 ml), it was evaporated to the dry state in a vacuum rotary evaporator. The residue was taken up in a mixture of water (150 mL) and ethyl acetate (150 mL). The organic phase was separated, washed with water, dried with anhydrous sodium sulfate and concentrated by evaporation in a VRV. The residue was recrystallized from acetone/n-hexane, whereby the title compound was obtained; melting point 194-196°C.

### Example 27

### ent-Estradiol-3-(N-propionyl)sulfamate

Triethylamine (0.4 mL), p-dimethylaminopyridine (0.35 g) and propionic acid anhydride (7.4 mL) were added in succession to a solution of ent-estrone-sulfamate (1.0 g) in dichloromethane (35 mL). The reaction mixture was stirred for 20 hours at +23°C, then it was decomposed with ice. The organic phase was washed with saturated aqueous sodium bicarbonate solution and water, dried on anhydrous sodium sulfate and concentrated by evaporation in a VRV, whereby ent-estrone-(N-propionyl)sulfamate was obtained; melting point 207-209°C. Reduction of this compound analogously to the reduction of estrone-(N-a cetyl)sulfamate yielded the title compound: melting point 199-202°C.

### Example 28

### ent-Estra-1,3,5(10)-triene-3,16β-diol

0.2 g (0.73 mmol) of ent-estra-1,3,5(10)-triene-3,16α-diol is dissolved in 20 ml of toluene with the addition of 0.77 g (2.94 mmol) of triphenylphosphine and 0.49 g (2.94 mmol) of 4-nitrobenzoic acid. 1.3 ml (2.94 mmol) of diethylazodicarboxylate solution (40% in toluene) is slowly added in drops to it. After a 48-hour reaction at room temperature, it is diluted with ethyl acetate, and the organic phase is washed with sodium bicarbonate solution, water and sodium chloride solution. It is dried on magnesium sulfate and concentrated by evaporation.

The product that is obtained (0.345 g) is dissolved in 50 ml of methanol and mixed with 1.67 g of potassium carbonate. It is stirred at room temperature until the saponification is completed. For working-up, the main amount of the methanol is distilled off, and the residue is taken up in ethyl acetate. It is washed with sodium chloride solution and dried on magnesium sulfate. After the concentration by evaporation, the crude product is chromatographed (silica gel; eluant: cyclohexane/ethyl acetate 2+1), whereby 0.17g (0.62 mmol; 85% of theory) of ent-estra-1,3,5(10)-triene-3,16β-diol accumulates. The recrystallization from ethyl acetate produces colorless crystals; melting point 235 ... 241°C; [α]_{D} = -76° (dioxane, c = 1.1).

### Example 29

### ent-7α-Methylestra-1,3,5(10)-triene-3,17β-diol

### 3,17-Bis(tetrahydropyran-2-yloxy)estra-1,3,5(10)-trien-6-one

0.25 g (0.87 mmol) of ent-3,17β-dihydroxyestra-1,3,5(10)-trien-6-one (cf. Example 6) is dissolved in 25 ml of methylene chloride and mixed with 1.58 ml of 3,4-dihydro-2H-pyran as well as 44 mg of pyridinium-4-toluenesulfonate. The solution is stirred at room temperature, and after the reaction is completed, it is mixed with 150 ml of saturated sodium bicarbonate solution. After extraction with methylene chloride, it is dried on magnesium sulfate and concentrated by evaporation. The crude product is purified on silica gel (eluant: cyclohexane/ethyl acetate 4+1), whereby 0.4 g (0.87 mmol; 99% of theory) of 3,17β-bis(tetrahydropyran-2-yloxy)estra-1,3,5(10)-trien-6-one accumulates as a colorless foam.

### 3,17β-Bis(tetrahydropyran-2-yloxy)-7α-methylestra-1,3,5(10)-trien-6-one

0.42 g (3.75 mmol) of potassium-tert-butylate in 3.5 ml of tert-butanol is dissolved in a moisture-free environment. 0.396 g (0.87 mmol) of 3,17-bis(tetrahydropyran-2-yloxy)estra-1,3,5(10)-trien-6-one, dissolved in 2 ml of 1,2-dimethoxy-ethane, is added to this solution. Then, it is mixed with 0.27 ml (4.3 mmol) of methyl iodide and stirred at room temperature until the reaction is completed. For working-up, it is mixed with saturated sodium chloride solution and extracted with ethyl acetate. The crude product is purified on silica gel (eluant: cyclohexane/ethyl acetate 8+1). 0.15 g (0.33 mmol; 37% of theory) of 3,17β-bis(tetrahydropyran-2-yloxy)-7α-methylestra-1,3,5(10)-trien-6-one is obtained as a colorless foam.

### ent-7α-Methylestra-1,3,5(10)-triene-3,17β-diol

150 mg (0.33 mmol) of 3,17β-bis(tetrahydropyran-2-ytoxy)-7α-methylestra-1,3,5(10)-trien-6-one is dissolved in 10 ml of methylene chloride and mixed with 3.1 ml (19.2 mmol) of triethylsilane. The solution is then cooled to -10°C. 6.43 ml (51.2 mmol) of boron trifluoride ethyl etherate is added, and it is stirred for 1 hour at -10°C. It is then mixed with saturated sodium chloride solution and extracted with ethyl acetate. After drying (MgSO₄) and evaporation of the solvent, 90 mg (3.14 mmol; 95% of theory) of ent-7α -methylestra-1,3,5(10)-triene-3,17 β-diol is obtained. Recrystallization from ethyl acetate yields colorless crystals; melting point 151...155°C; [α]_{D} = -54° (dioxane, c = 1.29).

### Example 30

### ent-11β-Fluoro-estra-1,3,5(10)-triene-3,17β-diol

### ent-3,17β-Bis-(tert-butyldimethyl)silyloxy-estra-1,3,5(10)-trien-11α-ol

First 13.5 g of imidazole and then 17.1 g of tert-butyldimethylchlorosilane were added to 4.5 g of ent-estra-1,3,5(10),9(11)-tetraene-3,17β-diol (Example 5) in 105 ml of dimethylformamide, and it was stirred for 1 hour at room temperature. The reaction mixture was mixed with 100 ml of n-hexane, washed several times with water and dried on sodium sulfate. The solvent was evaporated in a vacuum, and the foamy residue (8.5 g) was used in the next stage without further purification.

8.5 g of crude ent-3,17 β-bis-(tert-butyldimethyl)silyloxy-estra-1,3,5(10),9(11)-tetraene (2) was dissolved in 40 ml of THF, mixed carefully with 4.9 ml of borane-dimethyl sulfide complex at a temperature of 50°C, and the temperature was maintained for 1 hour. Then, 8 ml of water and 32 ml of 3N sodium hydroxide solution were added in succession at 0°C, and then mixed with 15.5 ml of 30% hydrogen peroxide. The reaction mixture was stirred for four hours while being heated to room temperature, then 200 ml of n-hexane was added; it was washed with saturated sodium thiosulfate solution and saturated sodium chloride solution and dried on sodium sulfate. The solvent was distilled off in a rotary evaporator, and the residue was chromatographed on silica gel (solvent mixture: cyclohexane/ethyl acetate = 96/4). The product ent-3,17β-bis-(tert-butyldimethyl)silyloxy-estra-1,3,5(10)-trien-11α-ol (3) accumulated as a foam in a yield of 5.6 g.

### ent-11β-Fluoro-3-perfluorobutanesulfonyloxy-17β-(tert-butyldimethyl)silyloxy. estra-1,3,5(10)-triene (4)

A solution of 550 mg of ent-3,17 β-bis-(tert-butyldimethyl)silyloxy-estra-1,3,5(10)-trien-11α -ol (3) in 4 ml of anhydrous toluene was mixed at -5°C with 0.5 ml of 1,8-diazabicyclo[5.4.0]undec-7-ene and slowly with 0.4 ml of perfluorobutanesulfonic acid fluoride. While being heated to room temperature, it was stirred for 1 hour. The mixture was taken up in dichloromethane, washed several times with water, dried on sodium sulfate and evaporated to the dry state in a vacuum. By chromatography of the residue on silica gel (solvent mixture: cyclohexane/ethyl acetate = 99/1), 511 mg of oily ent-11β-fluoro-3-perfluorobutane-sulfonyloxy-17β-(tert-butyldimethyl)silyloxy-estra-1,3,5(10)-triene (4) was obtained.

### ent-11β-Fluoro-estra-1,3,5(10)-triene-3,17β-diol (5)

374 mg of lithium aluminum hydride in 100 ml of anhydrous tetrahydrofuran was mixed at -78°C under argon with a solution of 420 mg of ent-11β-fluoro-3-perfluorobutanesulfonyloxy-17β-(tert-butyldimethyl)silyloxy-estra-1,3,5(10)-triene (4) in 20 ml of tetrahydrofuran, kept at this temperature for 10 minutes and then stirred for another 3 hours while being heated to room temperature. The mixture was mixed with water while being cooled with ice, then acidified with 4N sulfuric acid and extracted three times each with toluene and diethyl ether. After the collected organic phases were dried on sodium sulfate, it was evaporated to the dry state in a vacuum. The evaporation residue was dissolved in 9 ml of tetrahydrofuran, 3.90 g of tetrabutylammonium fluoride-trihydrate was added, and it was stirred for 3.5 hours at 50°C. Then, the mixture was cooled, acidified with 4N sulfuric acid to a pH of 2 and mixed with 10 ml each of water and ethyl acetate. The organic phase was separated, dried with sodium sulfate, the solvent was removed in a rotary evaporator, and the residue was chromatographed on silica gel (solvent mixture: toluene/ethyl acetate/chloroform = 6/3/1). 148 mg of colorless ent-11β-fluoro-estra-1,3,5(10)-triene-3,17β-diol (5) with a melting point of 199-200°C was obtained.

### Example 31

### ent-9α-Methyl-estra-1,3,5(10)-triene-3,17 β-triol

### ent-3,17 β-Bis-(tert-butyldimethyl)silyloxy-estra-1,3,5(14)-trien-11-one (6)

3.16 g of ent-3,17 β-bis-(tert-butyldimethyl)silyloxy-estra-1,3,5(10)-trien-11α-ol (Example 30) was dissolved in 30 ml of dichloromethane and stirred at room temperature with 4.1 g of pyridinium chlorochromate in 12 g of aluminum oxide for 3 hours. The reaction batch was filtered with dichloromethane on a little silica gel and then purified on silica gel (solvent mixture: dichloromethane/methanol = 3/1). 2.47 g of 11-oxosteroid 6 in the form of a colorless foam was obtained.

### ent-9α-Methyl-3,17β-bis-(tert-butyldimethyl)silyloxy-estra-1,3,5(10)-trien-11-one (7a)

890 mg of solid potassium-tert-butylate was added under argon at room temperature to a solution of 1.0 g of ent-3,17β-bis-(tert-butyldimethyl)silyloxy-estra-1,3,5(10)-trien-11-one (6) in 7.3 ml of methyl iodide and 7,3 ml of tert-butanol, and the mixture was stirred for 15 hours at room temperature. Then, it was poured onto crushed ice, extracted three times with dichloromethane and dried on sodium sulfate. The solvent was removed in a rotary evaporator, and the residue was purified on silica gel (solvent mixture: dichloromethane/cyclohexane = 1/1). 770 mg of steroid 7a in the form of a colorless foam was obtained.

### ent-9α-Methyl-estra-1,3,5(10)-triene-3,17β-diol (8a)

350 mg of ent-9α -methyl-3,17β-bis-(tert-butyldimethyl)silyloxy-estra-1,3,5(10)-trien-11-one (7a) was added to a solution of 930 mg of potassium hydroxide in 70 ml of triethylene glycol, mixed with 0.74 ml of hydrazinium hydrate (80% with water) and heated for 3 hours to 205°C. After cooling, it was diluted with 55 ml of water, 10.5 ml of 4N sulfuric acid was carefully added and extracted several times with diethyl ether. The collected organic phases were dried on sodium sulfate, concentrated by evaporation in a vacuum, and the crude product was recrystallized from n-hexanelacetone. ent-9α-Methyl-estra-1,3,5(10)-triene-3,17β-diol accumulated in a yield of 135 mg with a melting point of 144-147°C.

### Example 32

### ent-9a-Ethyi-estra-1,3,5(10)-triene-3,17 β-diol (8b)

Analogously to Example 31, 54 mg of ent-9α-ethyl-estra-1,3,5(10)-triene-3,17β-diol with a melting point of 121-123°C was obtained from 1.0 g of ent-3,17β-bis-(tert-butyldimethyl)silyloxy-estra-1,3,5(10)-trien-11-one.

### Example 33

### ent-14α,15α-Methylen-estra-1,3,5(10),8-tetraene-3,17α-diol

### ent-Methoxy-14α,15α-methylen-estra-1,3,5(10),8-tetraen-17α-ol

22.5 g of zinc dust, 5.0 g of ent-3-methoxy-estra-1,3,5(10),8,14-pentaen-17α-ol, and 30 mg of iodine were suspended in 50 ml of tetrahydrofuran and heated to a temperature of 70°C under argon. Then, 8 ml of diiodomethane was slowly added in drops under ultrasound (35 kHz), and the mixture was kept at 70°C for another 3 hours under ultrasound. Then, it was cooled to 10°C, diluted with 75 ml of ethyl acetate and decomposed by adding 370 ml of 20% ammonium chloride solution. It was filtered off from the precipitate and rewashed several times with ammonium chloride solution and water. In this case, the precipitate was kept constantly wet until its disposal. The organic phase of the filtrate was separated, and the aqueous phase was extracted several times with ethyl acetate. The collected organic phases were washed in succession with aqueous ammonium chloride solution, aqueous sodium thiosulfate solution and water and subjected to a subsequent steam distillation. The product was obtained by filtration of the suspension obtained in the distillation. The filter cake was dried first in the desiccator on calcium chloride and then recrystallized from methanol. 3.0 g of colorless crystals with a melting point of 167-168°C was obtained.

### ent-14α,15α-Methylen-estra-1,3,5(10),8-tetraene-3,17α-diol

ent-14α,15α-Methylen-estra-1,3,5(10),8-tetraene-3,17α-diol was produced by ether cleavage from ent-3-methoxy-14α,15α-methylen-estra-1,3,5(10),8-tetraen-17α-ol with DIBAH according to the general operating instructions. The melting point is not characteristic (decomposition T > 80°C).

### Example 34

### ent-14α,15α-Methylen-estra-1,3,5(10)-triene-3,17β-diol

### ent-3-Methoxy-14α,15α-methylen-estra-1,3,5(10)-trien-17α-ol

1.33 g of ent-3-methoxy-14α ,15α -methylen-estra-1,3,5(10),8-tetraen-17a -ol, dissolved in 20 ml of tetrahydrofuran, and 1.6 ml of aniline, were added in drops to 55 ml of liquid ammonia. 200 mg of lithium in small pieces was added successively at a temperature of -60°C. After the reduction was completed, 1.9 g of ammonium chloride was added to the reaction mixture, and the ammonia was evaporated. The residue was mixed with water and extracted with methylene chloride. The organic phase was washed neutral, dried and concentrated by evaporation. The crude product was recrystallized from methanol. In this case, 0.9 g of ent-3-methoxy-14α,15α-methylen-estra-1,3,5(10)-trien-17α-ol was isolated as colorless crystals.

### ent-3-Methoxy-14α,15α-methylen-estra-1,3,5(10)-trien-17β-ol

A solution of 0.9 g of ent-3-methoxy-14α,15α -methylen-estra-1,3,5(10)-trien-17a -ol in 25 ml of acetone was cooled to 10°C, and 1.7 ml of Jones reagent was added in drops within 6 minutes. After 0.25 hour, first 0.9 ml of isopropanol and then 35 ml of water were added and extracted several times with ethyl acetate. The collected organic phases were washed with saturated sodium bicarbonate solution and water, dried on sodium sulfate and evaporated to the dry state in a rotary evaporator. The crude product (0.9 g) was used in the next stage without further purification.

A solution of 0.9 g of crude ent-3-methoxy-14a,15α -methylen-estra-1,3,5(10)-trien-17-one in 3 ml of tetrahydrofuran was stirred for 3.5 hours at 0°C with a solution of diborane (prepared fresh from sodium borohydride and boron trifluoride etherate in tetrahydrofuran). After the reaction mixture was hydrolyzed with water, the precipitate that was produced was filtered off and dried in air. By chromatographic purification on silica gel (solvent mixture: cyclohexane/methyl-tert-butyl ether = 7/3), 511 mg of ent-3-methoxy-14α,15α-methylen-estra-1,3,5(10)trien-17β-ol was obtained in the form of a colorless foam.

### ent-14α,15α-Methylen-estra-1,3,5(10)-triene-3,17β-diol

ent-14α,15α-Methylen-estra-1,3,5(10)-triene-3,17β-diol was produced by ether cleavage of ent-3-methoxy-14α,15α-methylen-estra-1,3,5(10)-trien-17β-ol with DIBAH according to general operating instructions. The melting point is 213-215°C.

### Example 35

### ent-3,16α-Dihydroxy-estra-1,3,5(10)-trien-17-one

### ent-3-tert-Butyldimethylsilyloxy-estra-1,3,5(10)-trien-17-one

4.7 g (17.4 mmol) of ent-3-hydroxy-estra-1,3,5(10)-trien-17-one and 4.5 g (65.7 mmol) of imidazole are dissolved at room temperature in 100 ml of DMF. Then, the solution is mixed with 5.9 g (39.2 mmol) of tert-butyldimethylchlorosilane. The reaction temperature increases to about 30°C, and the solution turns dark brown. The educt has reacted completely to silyl ether after 1.5 hours. To isolate the product, the reaction solution in 250 ml of ice water is added in drops. The suspension that is obtained is suctioned off, washed with water, and the product is dried.

The crude product that is thus obtained is purified by filtration on silica gel (methylene chloride).

5.7 g (85% of theory) of ent-3-tert-butyldimethylsilyloxy-estra-1,3,5(10)-trien-17-one is obtained.

### ent-3-tert-Butyldimethylsilyloxy-17-trimethylsilyloxy-estra-1,3,5(10),16-tetraene

1.24 ml (8.8 mmol) of diisopropylamine is added to 10 ml of tetrahydrofuran and cooled to about -30°C. While being cooled steadily, 3.2 ml (8 mmol) of n-butyllithium solution (2.5 M in toluene) is added in drops. After 0.5 hour of reaction time at about -30°C, 769 mg (2 mmol) of ent-3-tert-butyldimethylsilyloxy-estra-1,3,5(10)-trien-17-one, dissolved in 5 ml of tetrahydrofuran, is added to the solution. Then, after another 0.5 hour of stirring, the mixture is mixed with 1.54 ml (12 mmol) of trimethylchlorosilane. It is stirred for one more hour at about -30°C, and the reaction solution is added in sodium bicarbonate solution. The extraction of the product is carried out with chloroform. After the extraction solution is concentrated by evaporation to a volume of about 10 ml, this solution is used immediately in the next stage.

### ent-3-tert-Butyldimethylsilyloxy-16a,17α-epoxy-17-trimethylsilyloxy-estra-1,3,5(10)-triene

740 mg (4.3 mmol) of m-chloroperbenzoic acid is added to the chloroform solution from the precursor (about 10 ml). The reaction temperature slightly increases. After one hour of stirring at room temperature, the reaction solution is washed with sodium bicarbonate solution and water. After drying on sodium sulfate and concentration by evaporation of the chloroform solution, it was possible to obtain 0.91 g of yellow, crystalline substance.

### ent-3,16α-Dihydroxy-estra-1,3,5(10)-trien-17-one

0.91 g of ent-3-tert-butyldimethylsilyloxy-16α,17α-epoxy-17-trimethylsilyloxy-estra-1,3,5(10)-triene (crude product) is dissolved in methanol, and the pH is set at 8-9 with aqueous sodium hydroxide solution (5 M). After several hours of stirring, the reaction solution is neutralized, methanol is distilled off, and the reaction products are extracted with ethyl acetate. The crude product is purified by chromatography on silica gel (cyclohexane/ethyl acetate, 2/1).
Yield: 175 mg (65% of theory relative to ent-3-hydroxy-estra-1,3,5(10)-trien-17-one)
Melting point 204-208°C: [α]²⁵_{D} = -170° (ethanol)

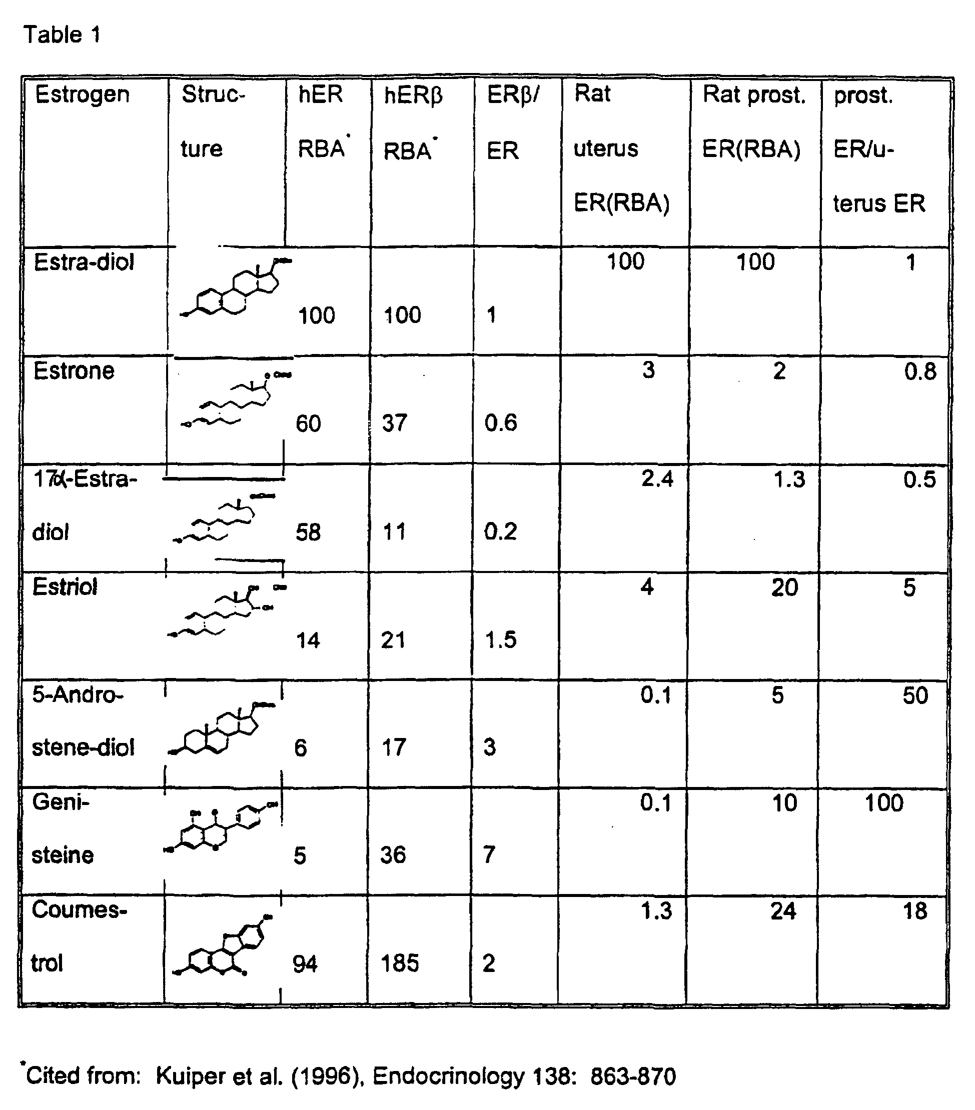

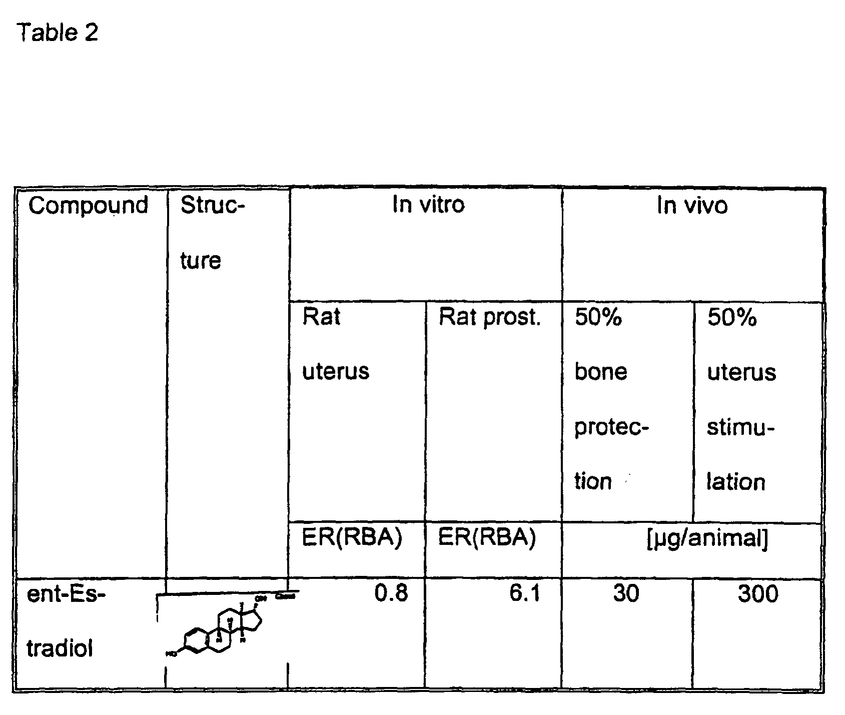

## Claims

1. Ent-Steroids namely
ent-Estriol
ent-Estriol-3-sulfamate
ent-Estriol-3-(N-acetyl)sulfamate
ent-Estriol-3,16,17-tripropionate
ent-Estrone-3-sulfamate
ent-Estrone-3-(N-acetyl)sulfamate
ent-Estradiol-3-sulfamate
ent-Estradiot-3,17-disulfamate
ent-Estradiol-3-(N-acetyl)sulfamate
ent-Estradiol-3,17-bis-(N-acetyl)sulfamate
ent-Estrone-(N-propionyl)sulfamate
ent-Estradiol-3-(N,N-dimethyl)sulfamate
ent-Estradiol-3-(N,N-diethyl)sulfamate
ent-Estradiol-3-pyrrolidinosulfonate
ent-Estradiol-17-valerianate
ent-Estradiol-17-decanoate
ent-3,17β-Dihydroxy-estra-1,3,5(10)-trien-2-yl-sulfamate
ent-16α-Hydroxy-17-oxo-estra-1,3,5(10)-trien-3-yl-sulfamate
ent-16α-Hydroxy-3-methoxy-estra-1,3,5 (10)-trien-17-one
ent-Estra-1,3,5(10)-triene-3,16α-diol
ent-3-Methoxy-estra-1,3,5(10)-triene-16α,17β-diol
ent-2-Methoxy-estra-1,3,5(10)-triene-3,17β-diol
ent-17β-Hydroxy-14α,15α-methylen-estra-1,3,5(10)-trien-3-yl-(N-acetyl)sulfamate
ent-14α,15α-Methylen-estra-1,3,5(10)-triene-3,17β-diol
ent-14β,15β-Methylen-estra-1,3,5(10)-triene-3,17β-diol
ent-14α,15α-Methylen-estra-1,3,5(10),8-tetraene-3,17α-diol
ent-17α-Hydroxy-14α,15α-methylen-estra-1,3,5(10),8-tetraen-3-yl-(N-acetyl)sulfamate
ent-14β,15β-Methylen-estra-1,3,5(10),8-tetraene-3,17β-diol
ent-16α-Hydroxy-estra-1,3,5(10)-trien-3-yl-sulfamate
ent-16α-Hydroxy-estra-1,3,5(10)-trien-3-yl-benzoate
ent-3-Hydroxy-estra-1,3,5(10)-trien-17α-yl-undecanoate
ent-17β-Hydroxy-estra-1,3,5(10),8-tetraen-3-yl-sulfamate
ent-17β-Hydroxy-estra-1,3,5(10),8-tetraen-3-yl-(N-butyryl)sulfamate
ent-Estra-1,3,5(10),8-tetraene-3,17α-diol
ent-Estra-1,3,5(10),8-tetraene-3,17β-diol
ent-Estra-1,3,5(10),9(11)-tetraene-3,17β-diol
ent-Estra-1,3,5(10),7-tetraene-3,17β-diol
ent-Estra-1,3,5(10),7-tetraene-3,17α-diol
ent-17β-Hydroxy-estra-1,3,5(10),7-tetraen-3-yl-sulfamate
ent-Estra-1,3,5(10),6-tetraene-3,17β-diol
ent-Estra-1,3,5(10),6,8-tetraene-3,17β-diol
ent-Estra-1,3,5(10),6,8-tetraene-3,17α-diol
ent-Estra-1,3,5(10),8,14-pentaene-3,17β-diol-3-butyrate
ent-Estra-1,3,5(10),8,14-pentaene-3,17α-diol
ent-7α-Methylestra-1,3,5(10)-triene-3,17β-diol
ent-9α-Methylestra-1,3,5(10)-triene-3,17β-diol
ent-9α-Ethylestra-1,3,5(10)-triene-3,17β-diol
ent-11β-Fluoroestra-1,3,5(10)-triene-3,17β-diol
ent-Estra-1,3,5(10)-triene-3,16β-diol

2. Use of ent-steroids of general formula I 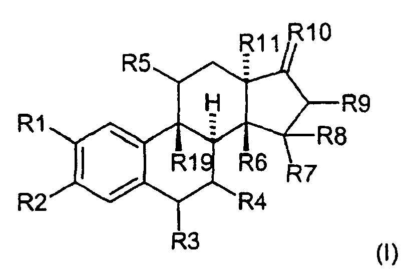 in which the term "aryl" as used herein means a phenyl, 1-, or 2-napthyl- or heteroaryl-radical, any of which can be substituted,
R¹ means a hydrogen atom;
a group R¹²-O-, whereby R¹² means a hydrogen atom or a hydrocarbon radical with up to 5 carbon atoms, which can contain a C-C-double bond or a C-C-triple bond;
a group R¹³SO₂-O-, in which R¹³ is an R¹⁴R¹⁵N group, whereby R¹⁴ and R¹⁵, independently of one another, mean a hydrogen atom, a C₁-C₅ alkyl radical, a group C(O)R¹⁶, in which R¹⁶ represents a hydrocarbon radical with up to 12 carbon atoms, which can additionally contain up to three double bonds and/or triple bonds, a C₃-C₇ cycloalkyl radical, an aryl radical or a combination thereof or, together with the N-atom mean a polymethylenimino radical with 4 to 6 C atoms or a morpholino radical;
R² means a group R¹²-O-, R¹³SO₂-O- or -O-C(O)R¹⁶, with R¹², R¹³ and R¹⁶ in each case in the meaning that is indicated under R¹;
R³, R⁴, R⁵, R⁸ and R⁹, independently of one another, mean a hydrogen atom, a halogen atom, a group R¹²-O-, R¹³SO₂-O-, or-R¹⁶, with R¹², R¹³ and R¹⁶ in each case in the meaning that is indicated under R¹;
R⁶ means a β-position hydrogen atom and
R⁷ means a hydrogen atom
or
R⁶ and R⁷ together mean an α- or β-methylene group;
R¹⁰ means two hydrogen atoms; two halogen atoms; a hydrogen atom and a halogen atom; a group =CR¹⁷R¹⁸, in which R¹⁷ and R¹⁸, represent a hydrogen atom and a halogen atom; a hydrogen atom and a group R¹²-O-; a hydrogen atom and a group R¹³SO₂-O-; a group R¹² and a group -O-C(O)R¹⁶; a group R¹² and a hydroxyl group; with R¹², R¹³ and R¹⁶ in each case in the meaning that is indicated under R¹; an oxygen atom;
R¹¹ means a hydrogen atom, a methyl or ethyl group;
R¹⁹ means a hydrogen atom or a hydrocarbon radical with up to five carbon atoms, which can be partially or completely fluorinated and which can contain a C-C double bond or a C-C triple bond;
one or more double bonds can be present in positions 6, 7; 7, 8; 8, 9; 9, 11; 11, 12; 8, 14; 14, 15 and 15, 16 for the manufacture of a medicament for treatment of peri- and postmenopausal symptoms.

3. Use of steroids according to claim 2, which are derived from ent-13-alkylgonane.

4. Use of steroids according to claim 2 with one or more additional double bonds in rings B, C and D of the steroid skeleton.

5. Use of steroids according to claim 4 with are or more double bonds in position 6, 7; 7, 8; 8, 9; 9, 11; 11, 12; 8, 14; 14, 15; 15, 16.

6. Use of 13-alkylgonanes according to claim 3, in which R² is a hydroxy group.

7. Use of ent-13-alkylgona-1,3,5(10)-trien-3-ols according to claim 6, in which R¹⁰ means a hydrogen atom and a hydroxyl group.

8. Use of ent-13-alkylgona-1,3,5(10)-trien-3-ols according to claim 6, in which R⁹ means a hydroxyl group.

9. Use of ent-13-alkylgona-1,3,5(10)-trien-3-ols according to claim 6, in which R⁹ stands for a hydroxyl group and R¹⁰ stands for a hydrogen atom and a hydroxyl group.

10. Use of ent-13-alkylgona-1,3,5(10)-trien-3-ols according to claim 6, in which R¹⁰ stands for an oxygen atom.

11. Use of ent-13-alkylgona-1,3,5(10)-trien-3-ols according to claim 8, in which R¹⁰ stands for an oxygen atom.

12. Use of ent-steroids according to claim 3, in which R¹¹ is a methyl group.

13. Use of ent-steroids according to claim 3, in which R¹¹ is an ethyl group.

14. Use of the ent-steroids below according to claim 2
ent-Estriol
ent-Estriol-3-sulfamate
ent-Estriol-3-(N-acetyl)sulfamate
ent-Estriol-3,16,17-tripropionate
ent-Estrone-3-sulfamate
ent-Estrone-3-(N-acetyl)sulfamate
ent-Estradiol-3-sulfamate
ent-Estradiol-3,17-disulfamate
ent-Estradiol-3-(N-acetyl)sulfamate
ent-Estradiol-3,17-bis(N-acetyl)sulfamate
ent-Estrone-(N-propionyl)sulfamate
ent-Estradiol-3-(N,N-dimethyl)sulfamate
ent-Estradiol-3-(N,N-diethyl)sulfamate
ent-Estradiol-3-pyrrolidinosulfonate
ent-Estradiol-17-valerianate
ent-Estradiol-17-decanoate
ent-3,17β-Dihydroxy-estra-1,3,5(10)-trien-2-yl-sulfamate
ent-16α-Hydroxy-17-oxo-estra-1,3.5(10)-trien-3-yl-sulfamate
ent-16α-Hydroxy-3-methoxy-estra-1,3,5(10)-trien-17-one
ent-Estra-1,3,5(10)-triene-3,16α -diol
ent-3-Methoxy-estra-1,3,5(10)-triene-16α,17β-diol
ent-2-Methoxy-estra-1,3,5(10)-triene-3,17β-diol
ent-17β-Hydroxy-14α,15α-methylen-estra-1,3,5(10)-trien-3-yl-(N-acetyl)sulfamate
ent-14α,15α-Methylen-estra-1,3,5(10)-triene-3,17β-diol
ent-14β,15β-Methylen-estra-1,3,5(10)-triene-3,17β-diol
ent-14α,15α-Methylen-estra-1,3,5(10),8-tetraene-3,17α-diol
ent-17α-Hydroxy-14α,15α-methylen-estra-1,3,5(10),8-tetraen-3-yl-(N-acetyl)suffamate
ent-14β,15β-Methyten-estra-1,3.5(10),8-tetraene-3,17β-diol
ent-16α-Hydroxy-estra-1,3,5(10)-trien-3-yl-sulfamate
ent-16α-Hydroxy-estra-1,3,5(10)-trien-3-yl-benzoate
ent-3-Hydroxy-estra-1,3,5(10)-trien-17α-yl-undecanoate
ent-17β-Hydroxy-estra-1,3,5(10),8-tetraen-3-yl-sulfamate
ent-17β-Hydroxy-estra-1,3,5(10),8-tetraen-3-yl-(N-butyryl)sulfamate ent-Estra-1,3,5(10),8-tetraene-3,17α-diol
ent-Estra-1,3,5(10),8-tetraene-3,17β-diol
ent-Estra-1,3,5(10),9(11)-tetraene-3,17β-diol
ent-Estra-1,3,5(10),7-tetraene-3,17β-diol
ent-Estra-1,3,5(10),7-tetraene-3,17α-diol
ent-17β-Hydroxy-estra-1,3,5(10),7-tetraen-3-yl-sulfamate
ent-Estra-1,3,5(10),6-tetraene-3,17β-diol
ent-Estra-1,3,5(10),6,8-tetraene-3,17β-diol
ent-Estra-1,3,5(10),6,8-tetraene-3,17α-diol
ent-Estra-1,3,5(10),8,14-pentaene-3,17β-diol-3-butyrate
ent-Estra-1,3,5(10),8,14-pentaene-3,17α-diol
ent-Estradiol
ent-Estradiol-3-acetate
ent-Estradiol-17-acetate
ent-Estradiol-diacetate
ent-Estradiol-3-benzoate
ent-17α-Ethinyl-estra-1,3,5(10)-triene-3,17-diol
ent-17α-Ethinyl-estra-1,3,5(10)-triene-3,17-diyl-diacetate
ent-3-Hydroxy-estra-1,3,5(10),7-tetraen-17-one
ent-3-Hydroxy-estra-1,3,5(10),6,8-pentaen-17-one
ent-16β-Bromo-3-methoxy-estra-1,3,5(10)-trien-17-one
ent-7α-Methylestra-1,3,5(10)-triene-3,17β-diol
ent-9α-Methylestra-1,3,5(10)-triene-3,17β-diol
ent-9α-Ethylestra-1,3,5(10)-triene-3,17β-diol
ent-11β-Fluoroestra-1,3,5(10)-triene-3,17β-diol
ent-Estra-1,3,5(10)-triene-3,16β-diol
ent-3,16α-Dihydroxyestra-1,3,5(10)-trien-17-one.

15. Use of a compound of formula defined according to claim 2 to 14 for the manufacture of a medicament for treatment of peri- and post-male-menopausal symptoms.

16. Use according to claim 2 for prevention and treatment of hot flashes, sleep disturbances, irritability, mood swings, incontinence, vaginal atrophy, and hormone-deficiency-induced emotional diseases.

17. Use according to claim 16 for prevention and treatment of diseases in the urogenital tract.

18. Use of a compound of formula defined according to claim 2 to 14 for the manufacture of a medicament for prevention and therapy of gastrointestinal diseases.

19. Use according to claim 18 for prevention and therapy of ulcers and hemorrhagic diatheses in the gastrointestinal tract.

20. Use according to claim 19 for prevention and therapy of neoplasias.

21. Use of a compound of formula defined according to claim 2 to 14 except the compounds : (8α,9β,13α,14β)-3-[2-(dimethylamino)-ethoxy]-estra-1,3,5(10)-trien-17-one; (8α,9β,13α,14β)-3-[2-(diethylamino)-ethoxy]-estra-1,3,5(10)-trien-17-one; (8α,9β,13α,14β)-3-[2-(dimethylamino)-ethoxy)-estra-1,3,5(10)-trien;(8α,9β,13α,14β)-3-[2-(diethylamino)-ethoxy]-estra-1,3,5(10)-trien and its chlorohydrate; (8α,9β,13α,14β,17α)-3-[2,(diethylamino),ethoxy]-estra-1,3,5(10)-trien-17-ol for in-vitro treatment of male infertility.

22. Use of a compoung of formula defined according to claim 2 to 14 except the compounds : (8α,9β,13α,14β)-3-[2-(dimethylamino)-ethoxy]-estra-1,3,5(10)-trien-17-one; (8α,9β,13α,14β)-3-[2-(diethylamino)-ethoxy]-estra-1,3,5(10)-trien-17-one; (8α,9β,13α,14β)-3-[2-(dimethylamino)-ethoxy)-estra-1,3,5(10)-trien;(8α,9β,13α,14β)-3-[2-(diethylamino)-ethoxy]-estra-1,3,5(10)-trien and its chlorohydrate; (8α,9β,13α,14β,17α)-3-[2,(diethylamino),ethoxy]-estra-1,3,5(10)-trien-17-ol for in-vivo treatment of male infertility.

23. Use of a compound of formula defined according to claim 2 to 14 except the compounds : (8α,9β,13α,14β)-3-[2-(dimethylamino)-ethoxy]-estra-1,3,5(10)-trien-17-one; (8α,9β,13α,14β)-3-[2-(diethylamino)-ethoxy]-estra-1,3,5(10)-trien-17-one; (8α,9β,13α,14β)-3-[2-(dimethylamino)-ethoxy)-estra-1,3,5(10)-trien;(8α,9β,13α,14β)-3-[2-(diethylamino)-ethoxy]-estra-1,3,5(10)-trien and its chlorohydrate; (8α,9β,13α,14β,17α)-3-[2,(diethylamino),ethoxy]-estra-1,3,5(10)-trien-17-ol for in-vitro treatment of female infertility.

24. Use of a compound of formula defined according to claim 2 to 14 except the compounds : (8α,9β,13α,14β)-3-[2-(dimethylamino)-ethoxy]-estra-1,3,5(10)-trien-17-one; (8α,9β,13α,14β)-3-[2-(diethylamino)-ethoxy]-estra-1,3,5(10)-trien-17-one; (8α,9β,13α,14β)-3-[2-(dimethylamino)-ethoxy)-estra-1,3,5(10)-trien;(8α,9β,13α,14β)-3-[2-(diethylamino)-ethoxy]-estra-1,3,5(10)-trien and its chlorohydrate; (8α,9β,13α,14β,17α)-3-[2,(diethylamino),ethoxy]-estra-1,3,5(10)-trien-17-ol for in-vivo treatment of female infertility

25. Use of a compound of formula defined according to claim 2 to 14 except the compounds : (8α,9β,13α,14β)-3-[2-(dimethylamino)-ethoxy]-estra-1,3,5(10)-trien-17-one; (8α,9β,13α,14β)-3-[2-(diethylamino)-ethoxy]-estra-1,3,5(10)-trien-17-one; (8α,9β,13α,14β)-3-[2-(dimethylamino)-ethoxy)-estra-1,3,5(10)-trien;(8α,9β,13α,14β)-3-[2-(diethylamino)-ethoxy]-estra-1,3,5(10)-trien and its chlorohydrate; (8α,9β,13α,14β,17α)-3-[2,(diethylamino),ethoxy]-estra-1,3,5(10)-trien-17-ol for hormone replacement therapy (HRT).

26. Use of a compound of formula defined according to claim 2 to 14 except the compounds : (8α,9β,13α,14β)-3-[2-(dimethylamino)-ethoxy]-estra-1,3,5(10)-trien-17-one; (8α,9β,13α,14β)-3-[2-(diethylamino)-ethoxy]-estra-1,3,5(10)-trien-17-one; (8α,9β,13α,14β)-3-[2-(dimethylamino)-ethoxy)-estra-1,3,5(10)-trien;(8α,9β,13α,14β)-3-[2-(diethylamino)-ethoxy]-estra-1,3,5(10)-trien and its chlorohydrate; (8α,9β,13α,14β,17α)-3-[2,(diethylamino),ethoxy]-estra-1,3,5(10)-trien-17-ol for the therapy of hormone-deficiency-induced symptoms in the case of ovarian dysfunction.

27. Use of a compound of formula defined according to claim 2 to 14 for the manufacture of a medicament for prophylaxis and therapy of a hormone-deficiency-induced bone mass loss.

28. Use according to claim 27 for prophylaxis and therapy of osteoporosis.

29. Use of a compound of formula defined according to claim 2 to 14 for the manufacture of a medicament for prevention and therapy of cardiovascular diseases.

30. Use of a compound of formula defined according to claim 2 to 14 for the manufacture of a medicament for prevention and treatment of vascular diseases.

31. Use according to claim 30 for prevention and treatment of arteriosclerosis.

32. Use according to claim 30 for prevention and treatment of neointimal hyperplasias.

33. Use of a compound of formula defined acording to claim 2 to 14 for the manufacture of a medicament for prevention and treatment of hormone-deficiency-induced neurodegenerative diseases.

34. Use of a compound of formula defined according to claim 2 to 14 for the manufacture of a medicament for prevention and treatment of Alzheimer's disease and hormone-deficiency-induced impairment of memory and learning capacity.

35. Use of a comformed of formula defined according to claim 2 to 14 for the manufacture of a medicament for prevention and treatment of benign prostate hyperplasia (BPH).

36. Pharmaceutical compositions that contain at least one compound according to claim 1 as well as a pharmaceutically compatible vehicle.

## Revendications

1. Ent-stéroïdes, à savoir:
l'ent-estriol
l'ent-estriol-3-sulfamate
l'ent-estriol-3-(N-acétyl)sulfamate
l'ent-estriol-3,16,17-tripropionate
l'ent-estrone-3-sulfamate
l'ent-estrone-3-(N-acétyl)sulfamate
l'ent-estradiol-3-sulfamate
l'ent-estradiol-3,17-disulfamate
l'ent-estradiol-3-(N-acétyl)sulfamate
l'ent-estradiol-3,17-bis-(N-acétyl)sulfamate
l'ent-estrone-(N-propionyl)sulfamate
l'ent-estradiol-3-(N,N-diméthyl)sulfamate
l'ent-estradiol-3-(N,N-diéthyl)sulfamate
l'ent-estradiol-3-pyrrolidinosulfonate
l'ent-estradiol-17-valérianate
l'ent-estradiol-17-décanoate
l'ent-3,17β-dihydroxy-estra-1,3,5(10)-trién-2-yl-sulfamate
l'ent-16α-hydroxy-17-oxo-estra-1,3, 5 (10)-trién-3-yl-sulfamate
la ent-16α-hydroxy-3-méthoxy-estra-1,3,5(10)-trién-17-one
l'ent-estra-1,3,5(10)-triène-3,16α-diol
l'ent-3-méthoxy-estra-1,3,5(10)-triène-16α,17β-diol
l'ent-2-méthoxy-estra-1,3,5(10)-triène-3,17β-diol
l'ent-17β-hydroxy-14α,15α-méthylène-estra-1,3,5(10)-trién-3-yl-(N-acétyl)sulfamate;
l'ent-14α, 15α-méthylène-estra-1,3, 5(10) -triène-3,17β-diol
l'ent-14β,15β-méthylène-estra-1,3,5(10)-triène-3,17β-diol
l'ent-14α,15α-méthylène-estra-1,3,5(10),8-tétraène-3,17α-diol
l'ent-27α-hydroxy-14α,15α-méthylène-estra-1,3,5(10),8-tétraén-3-yl-(N-acétyl)sulfamate
l' ent-14β,15β-méthylène-estra-1,3,5(10), 8-tétraène-3,17β-diol
l'ent-16α-hydroxy-estra-1,3,5(10)-trién-3-yl-sulfamate
l'ent-16α-hydroxy-estra-1,3,5(10)-trién-3-yl-benzoate
l'ent-3-hydroxy-estra-1,3,5(10)-trién-17α-yl-undécanoate
l'ent-17β-hydroxy-estra-1,3,5(10),8-tétraén-3-yl-sulfamate
l'ent-17β-hydroxy-estra-1,3,5(10),8-tétraén-3-yl-(N-butyryl)sulfamate
l'ent-estra-1,3,5(10),8-tétraène-3,17α-diol
l'ent-estra-1,3,5(10),8-tétraène-3,17β-diol
l'ent-estra-1,3,5(10),9(11)-tétraène-3,17β-diol
l'ent-estra-1,3,5(10),7-tétraène-3,17β-diol
l'ent-estra-1,3,5(10),7-tétraène-3,17α-diol
l'ent-17β-hydroxy-estra-1,3,5(10),7-tétraén-3-yl-sulfamate
l'ent-estra-1,3,5(10),6-tétraène-3,17β-diol
l'ent-estra-1,3,5(10),6,8-tétraène-3,17β-diol
l'ent-estra-1,3,5(10),6,8-tétraène-3,17α-diol
l'ent-estra-1,3,5(10),8,14-pentaène-3,17β-diol-3-butyrate
l'ent-estra-1,3,5(10),8,14-pentaène-3,17α-diol
l'ent-7α-méthylestra-1,3,5(10)-triène-3,17β-diol
l'ent-9α-méthylestra-1,3,5(10)-triène-3,17β-diol
l'ent-9α-éthylestra-1,3,5(10)-triène-3,17β-diol
l'ent-11β-fluoroestra-1,3,5(10)-triène-3,17β-diol
l'ent-estra-1,3,5(10)-triène-3,16β-diol.

2. Utilisation d'ent-stéroïdes de formule générale I 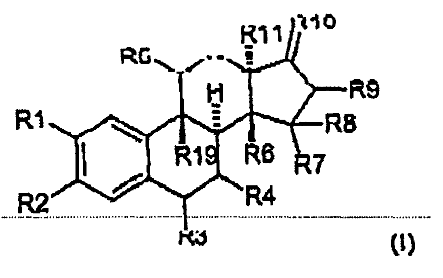
dans laquelle par le terme « aryle », tel qu'utilisé dans ce texte, on entend un radical phényle, 1- ou 2-naphtyle ou hétéroaryle, un quelconque de ceux-ci pouvant être substitué,
R¹ signifie un atome d'hydrogène ;
un groupement R¹²-O-, où R¹² signifie un atome d'hydrogène ou un radical hydrocarboné ayant jusqu'à 5 atomes de carbone, pouvant contenir une double liaison C-C ou une triple liaison C-C ;
un groupement R¹³SO₂-O-, où R¹³ est un groupement R¹⁴R¹⁵N, où R¹⁴ et R¹⁵, indépendamment l' un de l'autre, signifient un atome d'hydrogène, un radical alkyle en C₁-C₅, un groupement C(O)R¹⁶ , dans lequel R¹⁶ représente un radical hydrocarboné ayant jusqu'à 12 atomes de carbone, pouvant en outre contenir jusqu'à trois doubles liaisons et/ou triples liaisons, un radical cycloalkyle en C₃-C₇, un radical aryle ou une association de ceux-ci, ou, conjointement avec l'atome de N, signifient un radical polyméthylèneimino ayant de 4 à 6 atomes de C ou un radical morpholino ;
R² signifie un groupement R¹²-O-, R¹³ SO₂-O- ou -O-C (O) R¹⁶, R¹², R¹³ et R¹⁶ ayant dans chaque cas la signification qui est indiquée pour R¹
R³, R⁴, R⁵, R⁸ et R⁹, indépendamment les uns des autres, signifient un atome d'hydrogène, un atome d'halogène, un groupement R¹²-O-, R¹³-SO₂-O- ou -R¹⁶, R¹², R¹³ et R¹⁶ ayant dans chaque cas la signification qui est indiquée pour R¹ ;
R⁶ signifie un atome d' hydrogène en position β et
R⁷ signifie un atome d'hydrogène
ou
R⁶ et R⁷ signifient ensemble un groupement α- ou β-méthylène ;
R¹⁰ signifie deux atomes d'hydrogène ; deux atomes d'halogène ; un atome d'hydrogène et un atome d'halogène ; un groupement =CR¹⁷R¹⁸, dans lequel R¹⁷ et R¹⁸ représentent un atome d'hydrogène et un atome d'halogène ; un atome d'hydrogène et un groupement R¹²⁻O- ; un atome d'hydrogène et un groupement R¹³SO₂-O- ; un groupement R¹² et un groupement -O-C(O)R¹⁶ ; un groupement R¹² et un groupement hydroxyle ; R¹², R¹³ et R¹⁶ ayant dans chaque cas la signification qui est indiquée pour R¹; un atome d'oxygène ;
R¹¹ signifie un atome d'hydrogène, un groupement méthyle ou éthyle ;
R¹⁹ signifie un atome d'hydrogène ou un radical hydrocarboné ayant jusque cinq atomes de carbone, pouvant être partiellement ou totalement fluoré et pouvant contenir une double liaison C-C ou une triple liaison C-C ;
pour la fabrication d'un médicament destiné au traitement des symptômes de péri- et postménopause.

3. Utilisation de stéroïdes selon la revendication 2, qui sont dérivés d'ent-13-alkylgonane.

4. Utilisation de stéroïdes selon la revendication 2, ayant une ou plusieurs doubles liaisons supplémentaires dans les cycles B, C et D du squelette stéroïdien.

5. Utilisation de stéroïdes selon la revendication 4, ayant une ou plusieurs doubles liaisons en position 6,7 ; 7,8 ; 8,9 ; 9,11 ; 11,12 ; 8,14 ; 14,15 ; 15,16.

6. Utilisation de 13-alkylgonanes selon la revendication 3, dans laquelle R² est un groupement hydroxy.

7. Utilisation d'ent-13-alkylgona-1,3,5(10)trién-3-ols selon la revendication 6, dans laquelle R¹⁰ signifie un atome d'hydrogène et un groupement hydroxyle.

8. Utilisation d'ent-13-alkylgona-1,3,5(10)trién-3-ols selon la revendication 6, dans laquelle R⁹ signifie un groupement hydroxyle.

9. Utilisation d'ent-13-alkylgona-1,3,5(10)trién-3-ols selon la revendication 6, dans laquelle R⁹ représente un groupement hydroxyle et R¹⁰ représente un atome d'hydrogène et un groupement hydroxyle.

10. Utilisation d'ent-13-alkylgona-1,3,5(10)trién-3-ols selon la revendication 6, dans laquelle R¹⁰ représente un atome d'oxygène.

11. Utilisation d'ent-13-alkylgona-1,3,5(10)trién-3-ols selon la revendication 8, dans laquelle R¹⁰ représente un atome d'oxygène.

12. Utilisation d'ent-stéroïdes selon la revendication 3, dans laquelle R¹¹ est un groupement méthyle.

13. Utilisation d'ent-stéroïdes selon la revendication 3, dans laquelle R¹¹ est un groupement éthyle.

14. Utilisation des ent-stéroïdes ci-après selon la revendication 2,
l'ent-estriol
l'ent-estriol-3-sulfamate
l'ent-estriol-3-(N-acétyl)sulfamate
l'ent-estriol-3,16,17-tripropionate
l'ent-estrone-3-sulfamate
l'ent-estrone-3-(N-acétyl)sulfamate
l'ent-estradiol-3-sulfamate
l'ent-estradiol-3,17-disulfamate
l'ent-estradiol-3-(N-acétyl)sulfamate
l'ent-estradiol-3,17-bis-(N-acétyl)sulfamate
l'ent-estrone-(N-propionyl)sulfamate
l'ent-estradiol-3-(N,N-diméthyl)sulfamate
l'ent-estradiol-3-(N,N-diéthyl)sulfamate
l'ent-estradiol-3-pyrrolidinosulfonate
l'ent-estradiol-17-valérianate
l'ent-estradiol-17-décanoate
l'ent-3,17β-dihydroxy-estra-1,3,5(10)-trién-2-yl-sulfamate
l'ent-16α-hydroxy-17-oxo-estra-1,3,5 (10)-trién-3-yl-sulfamate
la ent-16α-hydroxy-3-méthoxy-estra-1,3,5(10)-trién-17-one
l'ent-estra-1,3,5(10)-triène-3,16α-diol
l'ent-3-méthoxy-estra-1,3,5(10)-triène-16α,17β-diol
l' ent-2-méthoxy-estra-1,3,5 (10) -triène-3,17β-diol
l'ent-17β-hydroxy-14α,15α-méthylène-estra-1,3,5(10)-trién-3-yl-(N-acétyl)sulfamate
l'ent-14α,15α-méthylène-estra-1,3,5(10)-triène-3,17β-diol
l'ent-14β,15β-méthylène-estra-1,3,5(10)-triène-3,17β-diol
l'ent-14α,15α-méthylène-estra-1,3,5(10),8-tétraène-3,17α-diol
l'ent-17α-hydroxy-14α,15α-méthylène-estra-1,3,5(10),8-tétraén-3-yl-(N-acétyl)sulfamate
l'ent-14β,15β-méthylène-estra-1,3,5(10),8-tétraène-3,17β-diol
l'ent-16α-hydroxy-estra-1,3,5(10)-trién-3-yl-sulfamate
l'ent-16α-hydroxy-estra-1,3,5(10)-trién-3-yl-benzoate
l'ent-3-hydroxy-estra-1,3,5(10)-trién-17α-yl-umdécanoate
l'ent-17β-hydroxy-estra-1,3,5 (10),8-tétraén-3-yl-sulfamate
l'ent-17β-hydroxy-estra-1,3,5(10),8-tétraène-3-yl-(N-butyryl)sulfamate
l'ent-estra-1,3,5(10),8-tétraène-3,17α-diol
l'ent-estra-1,3,5(10),8-tétraène-3,17β-diol
l'ent-estra-1,3,5(10),9(11)-tétraène-3,17β-diol
l'ent-estra-1,3,5(10),7-tétraène-3,17β-diol
l'ent-estra-1,3,5(10),7-tétraène-3,27α-diol
l'ent-17β-hydroxy-estra-1,3,5(10),7-tétraén-3-yl-sulfamate
l'ent-estra-1,3,5(10),6-tétraène-3,17β-diol
l'ent-estra-1,3,5(10),6,8-tétraène-3,17β-diol
l'ent-estra-1,3,5(10),6,8-tétraène-3,17α-diol
l'ent-estra-1,3,5(10),8,14-pentaène-3,17β-diol-3-butyrate
l'ent-estra-1,3,5(10),8,14-pentaène-3,17α-diol
l'ent-estradiol
l'ent-estradiol-3-acétate
l'ent-estradiol-17-acétate
l'ent-estradiol-diacétate
l'ent-estradiol-3-benzoate
l'ent-17α-éthinyl-estra-1,3,5(10)-triène-3,17-diol
l'ent-17α-éthinyl-estra-1,3,5(10)-triène-3,17-diyl-diacétate
la ent-3-hydroxy-estra-1,3,5(10),7-tétraén-17-one
la ent-3-hydroxy-estra-1,3,5(10),6,8-pentaén-17-one
la ent-16β-bromo-3-méthoxy-estra-1,3,5(10-trién-17-one
l'ent-7α-méthylestra-1,3,5(10)-triène-3,17β-diol
l'ent-9α-méthylestra-1,3,5(10)-triène-3,17β-diol
l'ent-9α-éthylestra-1,3,5(10)-triène-3,17β-diol
l'ent-11β-fluoroestra-1,3,5(10)-triène-3,17β-diol
l'ent-estra-1,3,5(10)-triène-3,16β-diol
la ent-3,16α-dihydroxyestra-1,3,5(10)trién-17-one.

15. Utilisation d'un composé de formule définie selon les revendications 2 à 14, pour la fabrication d'un médicament destiné au traitement des symptômes de péri- et post andropause.

16. Utilisation selon la revendication 2, pour la prévention et le traitement des bouffées de chaleur, des troubles du sommeil, de l'irritabilité, des sautes d'humeur, de l'incontinence, de l'atrophie vaginale et des maladies émotionnelles induites par une déficience hormonale.

17. Utilisation selon la revendication 16, pour la prévention et le traitement de maladies du tractus urogénital.

18. Utilisation d'un composé de formule définie selon les revendications 2 à 14, pour la fabrication d'un médicament destiné à la prévention et la thérapie de maladies gastro-intestinales.

19. Utilisation selon la revendication 18, pour la prévention et la thérapie d'ulcères et de diathèses hémorragiques dans le tractus gastro-intestinal.

20. Utilisation selon la revendication 19, pour la prévention et la thérapie de néoplasies.

21. Utilisation d'un composé de formule définie selon les revendications 2 à 14, excepté les composés : la (8,9β,13α,14β)-3-[2-(diméthylamino)éthoxy]estra-1,3,5(10)-trién-17-one ; la (8α,9β,13α,14β)-3-[2-(diéthylamino)éthoxy]estra-1,3,5(10)-trién-17-one ; le (8α,9β,13α,14β)-3-[2-(diméthylamino)éthoxy]estra-1,3,5(10)-triène ; le (8α,9β,13α,14β)-3-[2-(diéthylamino)éthoxy]estra-1,3,5(10)-triène et son chlorhydrate ; le (8α,9β,13α,14β,17α)-3-[2-(diéthylamino)éthoxy]estra-1,3,5(10)-trién-17-ol ; pour la fabrication d'un médicament destiné au traitement *in vitro* de l'infertilité masculine.

22. Utilisation d'un composé de formule définie selon les revendications 2 à 14, excepté les composés ; la (8α,9β,13α,14β)-3-[2-(diméthylamino) éthoxy] estra-1,3,5(10)-trién-17-one ; la (8α,9β,13α,14β)-3-[2-(diéthylamino)éthoxy]estra-1,3,5(10)-trién-17-one ; le (8α,9β,13α,14β)-3-[2-(diméthylamino)éthoxy]estra-1,3,5(10)-triène ; le (8α,9β,13α,14β)-3-[2-(diéthylamino)éthoxy]estra-1,3,5(10)-triène et son chlorhydrate ; le (8α,9β,13α,14β,17α)-3-[2-(diéthylamino)-éthoxy]estra-1,3,5(10)-trién-17-ol ; pour la fabrication d'un médicament destiné au traitement in vivo de l'infertilité masculine.

23. Utilisation d'un composé de formule définie selon les revendications 2 à 14, pour la fabrication d'un médicament destiné au traitement *in vitro* de l'infertilité féminine.

24. Utilisation d'un composé de formule définie selon les revendications 2 à 14, pour la fabrication d'un médicament destiné au traitement *in vivo* de l'infertilité féminine.

25. Utilisation d'un composé de formule définie selon les revendications 2 à 14, pour la fabrication d'un médicament destiné à une hormonothérapie substitutive (HTR).

26. Utilisation d'un composé de formule définie selon les revendications 2 à 14, pour la fabrication d'un médicament destiné à la thérapie de symptômes induits par une déficience hormonale dans le cas d'un dysfonctionnement ovarien.

27. Utilisation d'un composé de formule définie selon les revendications 2 à 14, pour la fabrication d'un médicament destiné à la prophylaxie et la thérapie d'une perte de masse osseuse induite par une déficience hormonale.

28. Utilisation selon la revendication 27 , pour la prophylaxie et la thérapie de l'ostéoporose.

29. Utilisation d'un composé de formule définie selon les revendications 2 à 14, pour la fabrication d'un médicament destiné à la prévention et la thérapie de maladies cardiovasculaires.

30. Utilisation d'un composé de formule définie selon les revendications 2 à 14, pour la fabrication d'un médicament destiné à la prévention et au traitement de maladies vasculaires.

31. Utilisation selon la revendication 30, pour la prévention et le traitement de l'artériosclérose.

32. Utilisation selon la revendication 30, pour la prévention et le traitement des hyperplasies néointimales.

33. Utilisation d'un composé de formule définie selon les revendications 2 à 14, pour la fabrication d'un médicament destiné à la prévention et au traitement des maladies neurodégénératives induites par une déficience hormonale.

34. Utilisation d'un composé de formule définie selon les revendications 2 à 14, pour la fabrication d'un médicament destiné à la prévention et au traitement de la maladie d'Alzheimer et de la déficience de la mémoire et de la capacité d'apprentissage induite par une déficience hormonale.

35. Utilisation d'un composé de formule définie selon les revendications 2 à 14, pour la fabrication d'un médicament destiné à la prévention et au traitement de l'hypertrophie bénigne de la prostate (HBP).

36. Compositions pharmaceutiques contenant au moins un composé selon la revendication 1 ainsi qu'un véhicule pharmaceutiquement compatible.

## Patentansprüche

1. Verbindungen der allgemeinen Formel I, nämlich
ent-Estriol
ent-Estriol-3-sulfamat
ent-Estriol-3-(N-acetyl)sulfamat
ent-Estriol-3,16,17-tripropionat
ent-Estron-3-sulfamat
ent-Estron-3-(N-acetyl)sulfamat
ent-Estradiol-3-sulfamat
ent-Estradiol-3,17-disulfamat
ent-Estradiol-3-(N-acetyl)sulfamat
ent-Estradiol-3,17-bis-(N-acetyl)sulfamat
ent-Estron-(N-propionyl)sulfamat
ent-Estradiol-3-(N,N-dimethyl)sulfamat
ent-Estradiol-3-(N,N-diethyl)sulfamat
ent-Estradiol-3-pyrrolidinosulfonat
ent-Estradiol-17-valerianat
ent-Estradiol-17-decanoat
ent-3,17β-Dihydroxy-estra-1,3,5(10)-trien-2-yl-sulfamat
ent-16α-Hydroxy-17-oxo-estra-1,3,5(10)-trien-3-yl-sulfamat
ent-16α-Hydroxy-3-methoxy-esta-1,3,5(10)-trien-17-on
ent-Estra-1,3,5(10)-trien-3,16α-diol
ent-3-Methoxy-estra-1,3,5(10)-trien-3,16α,17β-triol
ent-2-Methoxy-estra-1,3,5(10)-trien-3,17β-diol
ent-17β-Hydroxy-14α,15α-methylen-estra-1,3,5(10)-then-3-yl-(N-acetyl)sulfamat
ent-14α,15α-Methylen-estra-1,3,5(10)-trien-3,17β-diol
ent-14β,15β-Methylen-estra-1,3,5(10)-trien-3,17β-diol
ent-14α,15α-Methylen-estra-1,3,5(10),8-tetraen-3,17α-diol
ent-17α-Hydroxy-14α,15α-methylen-estra-1,3,5(10),8-tetraen-3-yl-(N-acetyl)sulfamat
ent-14β,15β-Methylen-estra-1,3,5(10),8-tetraen-3,17β-diol
ent-16α-Hydroxy-estra-1,3,5(10)-trien-3-yl-sulfamat
ent-16α-Hydroxy-estra-1,3,5(10)-trien-3-yl-benzoat
ent-3-Hydroxy-estra-1,3,5(10)-trien-17α-yl-undecanoat
ent-17β-Hydroxy-estra-1,3,5(10),8-tetraen-3-yl-sulfamat
ent-17β-Hydroxy-estra-1,3,5(10),8-tetraen-3-yl-(N-butyryl)sulfamat
ent-Estra-1,3,5(10),8-tetraen-3,17α-diol
ent-Estra-1,3,5(10),8-tetraen-3,17β-diol
ent-Estra-1,3,5(10),9(11)-tetraen-3,17β-diol
ent-Estra-1,3,5(10),7-tetraen-3,17β-diol
ent-Estra-1,3,5(10),7-tetraen-3,17α-diol
ent-17β-Hydroxy-estra-1,3,5(10),7-tetraen-3-yl-sulfamat
ent-Estra-1,3,5(10),6-tetraen-3,17β-diol
ent-Estra-1,3,5(10),6,8-tetraen-3,17β-diol
ent-Estra-1,3,5(10),6,8-tetraen-3,17α-diol
ent-Estra-1,3,5(10),8,14-pentaen-3,17β-diol-3-butyrat
ent-Estra-1,3,5(10),8,14-pentaen-3,17α-diol
ent-7α-Methylestra-1,3,5(10)-trien-3,17β-diol
ent-9α-Methylestra-1,3,5(10)-trien-3,17β-diol
ent-9α-Ethylestra-1,3,5(10)-trien-3,17β-diol
ent-11β-Fluoroestra-1,3,5(10)-trien-3,17β-diol
ent-Estra-1,3,5(10)-trien-3,16β-diol

2. Verwendung von ent-Steroiden der allgemeinen Formel I 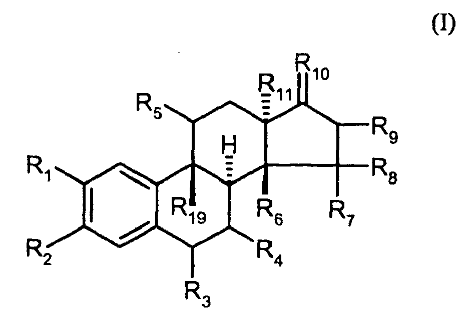
worin es sich bei dem Begriff "Aryl" um einen Phenyl-, 1- oder 2-Naphthyl oder Heteroarylrest handelt, von dem jeder substituiert sein kann,
R¹ ein Wasserstoffatom;
eine Gruppe R¹²-O-, wobei R¹² ein Wasserstoffatom oder einen Kohlenwasserstoffrest mit bis zu 5 Kohlenstoffatomen, der ein C-C Doppelbindungen oder ein C-C Dreifachbindung enthalten kann;
eine Gruppe R¹³SO₂-O-, worin R¹³ eine R¹⁴R¹⁵N - Gruppe ist, wobei R¹⁴ und R¹⁵ unabhängig voneinander ein Wasserstoffatom, einen C₁ - C₅ - Alkylrest, eine Gruppe C(O)R¹⁶, worin R¹⁶ einen Kohlenwasserstoffrest mit bis zu 12 Kohlenstoffatom, der außerdem bis zu drei Doppel- und/oder Dreifachbindungen enthalten kann, einen C₃-C₇₋Cycloalkylrest, einen Arylrest oder eine Kombination davon, oder, zusammen mit dem N-Atom, einen Polymethyleniminorest mit 4 bis 6 C-Atomen oder einen Morpholinorest, bedeuten;
R² eine Gruppe R¹²-O-, R¹³SO₂-O- oder-O-C(O)R¹⁶, mit R¹², R¹³ und R¹⁶ jeweils in der unter R¹ angegebenen Bedeutung;
R³, R⁴, R⁵, R⁸ und R⁹ unabhängig voneinander ein Wasserstoffatom, ein Halogenatom, eine Gruppe R¹²-O-, R¹³SO₂-O- oder -R¹⁶, mit R¹², R¹³ und R¹⁶ jeweils in der unter R¹ angegebenen Bedeutung;
R⁶ ein β-ständiges Wasserstoffatom und
R⁷ ein Wasserstoffatom
oder
R⁶ und R⁷ zusammen eine α- oder β-Methylengruppe;
R¹⁰ zwei Wasserstoffatome; zwei Halogenatome; ein Wasserstoffatom und ein Halogenatom; eine Gruppe =CR¹⁷R¹⁸, worin R¹⁷ und R¹⁸ ein Wasserstoffatom und ein Halogenatom darstellen; ein Wasserstoffatom und eine Hydroxylgruppe; ein Wasserstoffatom und eine Gruppe R¹²-O-; ein Wasserstoffatom und eine Gruppe R¹³SO₂-O-; eine Gruppe R¹² und eine Gruppe -O-C(O)R¹⁶; mit R¹², R¹³ und R¹⁶ jeweils in der unter R¹ angegebenen Bedeutung; eine Hydroxylgruppe und einen C₁-C₅₋Alkylrest; ein Sauerstoffatom;
R¹¹ ein Wasserstoffatom, eine Methyl- oder Ethylgruppe;
R¹⁹ ein Wasserstoffatom oder einen Kohlenwasserstoffrest mit bis zu 5 Kohlenstoffatomen, der eine C-C Doppelbindung oder eine C-C Dreifachbindung enthalten kann,
bedeuten
zur Herstellung eines Arzneimittels zur Behandlung von peri- und postmenopausalen Beschwerden.

3. Verwendung von Steroiden nach Anspruch 2, die sich vom ent-13-Alkylgonan ableiten.

4. Verwendung von Steroiden nach Anspruch 2 mit einer oder mehreren weiteren Doppelbindungen in den Ringen B, C, D des Steroidgerüsts.

5. Verwendung von Steroiden nach Anspruch 4 mit einer oder mehreren weiteren Doppelbindungen in den Positionen 6, 7; 7, 8; 8, 9; 9, 11; 11, 12; 8, 14; 14, 15; 15, 16.

6. Verwendung von 13-Alkylgonane nach Anspruch 3, worin R² eine Hydroxygruppe ist.

7. Verwendung von ent-13-Alkylgona-1,3,5(10)-trien-3-olen nach Anspruch 6, worin R¹⁰ ein Wasserstoffatom und eine Hydroxylgruppe bedeuten.

8. Verwendung von ent-13-Alkylgona-1,3,5(10)-trien-3-olen nach Anspruch 6, worin R⁸ eine Hydroxylgruppe bedeutet.

9. Verwendung von ent-13-Alkylgona-1,3,5(10)-trien-3-olen nach Anspruch 6, worin R⁸ für eine Hydroxylgruppe und R¹⁰ für ein Wasserstoffatom und eine Hydroxylgruppe stehen.

10. Verwendung von ent-13-Alkylgona-1,3,5(10)-trien-3-olen nach Anspruch 6, worin R¹⁰ für ein Sauerstoffatom steht.

11. Verwendung von ent-13-Alkylgona-1,3,5(10)-trien-3-olen nach Anspruch 8, worin R¹⁰ für ein Sauerstoffatom steht.

12. Verwendung von ent-Steroiden nach Anspruch 3, worin R¹¹ eine Methylgruppe ist.

13. Verwendung von ent-Steroiden nach Anspruch 3, worin R¹¹ eine Ethylgruppe ist.

14. Verwendung der nachstehenden ent-Steroide nach Anspruch 2 nämlich
ent-Estriol
ent-Estriol-3-sulfamat
ent-Estriol-3-(N-acetyl)sulfamat
ent-Estriol-3,16,17-tripropionat
ent-Estron-3-sulfamat
ent-Estron-3-(N-acetyl)sulfamat
ent-Estradiol-3-sulfamat
ent-Estradiol-3,17-disulfamat
ent-Estradiol-3-(N-acetyl)sulfamat
ent-Estradiol-3,17-bis-(N-acetyl)sulfamat
ent-Estron-(N-propionyl)sulfamat
ent-Estradiol-3-(N,N-dimethyl)sulfamat
ent-Estradiol-3-(N,N-diethyl)sulfamat
ent-Estradiol-3-pyrrolidinosulfonat
ent-Estradiol-17-valerianat
ent-Estradiol-17-decanoat
ent-3,17β-Dihydroxy-estra-1,3,5(10)-trien-2-yl-sulfamat
ent-16α-Hydroxy-17-oxo-estra-1,3,5(10)-trien-3-yl-sulfamat
ent-16α-Hydroxy-3-methoxy-estra-1,3,5(10)-trien-17-on
ent-Estra-1,3,5(10)-trien-3,16α-diol
ent-3-Methoxy-estra-1,3,5(10)-trien-3,16α,17β-triol
ent-2-Methoxy-estra-1,3,5(10)-trien-3,17β-diol
ent-17β-Hydroxy-14α,15α-methylen-estra-1,3,5(10)-trien-3-yl-(N-acetyl)sulfamat
ent-14α,15α-Methylen-estra-1,3,5(10)-trien-3,17β-diol
ent-14β,15β-Methylen-estra-1,3,5(10)-trien-3,17β-diol
ent-14α,15α-Methylen-estra-1,3,5(10),8-tetraen-3,17α-diol
ent-17α-Hydroxy-14α,15α-methylen-estra-1,3,5(10),8-tetraen-3-yl-(N-acetyl)sulfamat
ent-14β,15β-Methylen-estra-1,3,5(10),8-tetraen-3,17β-diol
ent-16α-Hydroxy-estra-1,3,5(10)-trien-3-yl-sulfamat
ent-16α-Hydroxy-estra-1,3,5(10)-trien-3-yl-benzoat
ent-3-Hydroxy-estra-1,3,5(10)-trien-17a-yl-undecanoat
ent-17β-Hydroxy-estra-1,3,5(10),8-tetraen-3-yl-sulfamat
ent-17β-Hydroxy-estra-1,3,5(10),8-tetraen-3-yl-(N-butyryl)sulfamat
ent-Estra-1,3,5(10),8-tetraen-3,17α-diol
ent-Estra-1,3,5(10),8-tetraen-3,17β-diol
ent-Estra-1,3,5(10),9(11)-tetraen-3,17β-diol
ent-Estra-1,3,5(10),7-tetraen-3,17β-diol
ent-Estra-1,3,5(10),7-tetraen-3,17α-diol
ent-17β-Hydroxy-estra-1,3,5(10),7-tetraen-3-yl-sulfamat
ent-Estra-1,3,5 (10),6-tetraen-3,17β-diol
ent-Estra-1,3,5(10),6,8-tetraen-3,17β-diol
ent-Estra-1,3,5(10),6,8-tetraen-3,17α-diol
ent-Estra-1,3,5( 10),8,14-pentaen-3,17β-diol-3-butyrat
ent-Estra-1,3,5(10),8,14-pentaen-3,17α-diol
ent-Estradiol
ent-Estradiol-3-acetat
ent-Estradiol-17-acetat
ent-Estradiol-diacetat
ent-Estradiol-3-benzoat
ent-17α-Ethinyl-estra-1,3,5(10)-trien-3,17-diol
ent-17α-Ethinyl-estra-1,3,5 (10)-trien-3,17-diyl-diacetat
ent-3-Hydroxy-estra-1,3,5 (10),7-tetraen-17-on
ent-3-Hydroxy-estra-1,3,5 (10),6,8-pentaen-17-on
ent-16β-Brom-3-methoxy-estra-1,3,5 (10)-trien-17-on
ent-7α-Methylestra-1,3,5 (10)-trien-3,17 β-diol
ent-9α-Methylestra-1,3,5 (10)-trien-3,17β-diol
ent-9α-Ethylesta-1,3,5(10)-trien-3,17β-diol
ent-1β-Fluoroestra-1,3,5(10)-trien-3,17β-diol
ent-Estra-1,3,5 (10)-trien-3,16β-diol
ent-3,16α-Dihydroxyestra-1,3,5(10)-trien-17-on

15. Verwendung einer Verbindung der Formeln nach Anspruch 2 bis 14 zur Herstellung eines Arzneimittels zur Behandlung von peri- und postandropausalen Beschwerden.

16. Verwendung nach Anspruch 2 zur Vorbeugung gegen und Behandlung von Hitzewallungen, Schlafstörungen, Reizbarkeit, Stimmungsschwankungen, Inkontinenz, Vaginalatrophie und hormondefizienzbedingter Gemütserkrankungen.

17. Verwendung nach Anspruch 16 zur Vorbeugung und Behandlung von Erkrankungen im Urogenitaltrakt.

18. Verwendung einer Verbindung der Formeln nach Anspruch 2 bis 14 zur Herstellung eines Arzneimittels zur Vorbeugung und Therapie von Magen- und Darmerkrankungen.

19. Verwendung nach Anspruch 18 zur Vorbeugung und Therapie von Ulcera und hämoragischen Diathesen im Magendarmtrakt.

20. Verwendung nach Anspruch 19 zur Vorbeugung und Therapie von Neoplasien.

21. Verwendung einer Verbindung der Formeln nach Anspruch 2 bis 14 zur Herstellung eines Arzneimittels ausgenommen der Verbindungen (8α,9β,13α,14β)-3-[2-(dimethylamino)-ethoxy]-estra-1,3,5(10)-trien-17-on; (8α,9β,13α,14β)-3-[2-(diethylamino)-ethoxy]-estra-1,3,5(10)-trien-17-on; (8α,9β,13α,14β)-3-[2-(dimethylamino)-ethoxy]-estra-1,3,5(10)-trien; (8α,9β,13α,14β)-3-[2-(diethylamino)-ethoxy]-estra-1,3,5(10)-trien und deren Chloridrat; (8α,9β,13α,14β,17α)-3-[2-(diethylamino)-ethoxy]-estra-1,3,5(10)-trien-17-ol für die in-vitro Behandlung der männlichen Infertilität.

22. Verwendung einer Verbindung der Formeln nach Anspruch 2 bis 14 zur Herstellung eines Arzneimittels ausgenommen der Verbindungen (8α,9β,13α,14β)-3-[2-(dimethylamino)-ethoxy]-estra-1,3,5(10)-trien-17-on; (8α,9β,13α,14β)-3-[2-(diethylamino)-ethoxy]-estra-1,3,5(10)-trien-17-on; (8α,9β,13α,14β)-3-[2-(dimethylamino)-ethoxy]-estra-1,3,5(10)-trien; (8α,9β,13α,14β)-3-[2-(diethylamino)-ethoxy]-estra-1,3,5(10)-trien und deren Chloridrat; (8α,9β,13α,14β,17α)-3-[2-(diethylamino)-ethoxy]-estra-1,3,5(10)-trien-17-ol für die in-vivo Behandlung der männlichen Infertilität.

23. Verwendung einer Verbindung der Formeln nach Anspruch 2 bis 14 zur Herstellung eines Arzneimittels für die in-vitro Behandlung der weiblichen Infertilität.

24. Verwendung einer Verbindung der Formeln nach Anspruch 2 bis 14 zur Herstellung eines Arzneimittels für die in-vivo Behandlung der weiblichen Infertilität.

25. Verwendung einer Verbindung der Formeln nach Anspruch 2 bis 14 zur Herstellung eines Arzneimittels für die Hormonersatz-Therapie (HRT).

26. Verwendung nach einer Verbindung der Formeln nach Anspruch 2 bis 14 zur Herstellung eines Arzneimittels für die Therapie von hormondefizienz bedingten Beschwerden bei ovarieller Dysfunktion.

27. Verwendung einer Verbindung der Formeln nach Anspruch 2 bis 14 zur Herstellung eines Arzneimittels zur Prophylaxe und Therapie eines hormondefizienzbedingten Knochenmasseverlustes.

28. Verwendung nach Anspruch 27 zur Prophylaxe und Therapie der Osteoporose.

29. Verwendung einer Verbindung der Formeln nach Anspruch 2 bis 14 zur Herstellung eines Arzneimittels zur Vorbeugung gegen und Therapie von Herzkreislauferkrankungen.

30. Verwendung einer Verbindung der Formeln nach Anspruch 2 bis 14 zur Herstellung eines Arzneimittels zur Vorbeugung gegen und Behandlung von Gefäßerkrankungen.

31. Verwendung nach Anspruch 30 zur Vorbeugung gegen und Behandlung von Atherosklerose.

32. Verwendung nach Anspruch 30 zur Vorbeugung und Behandlung neointimaler Hyperplasien.

33. Verwendung einer Verbindung der Formeln nach Anspruch 2 bis 14 zur Herstellung eines Arzneimittels zur Vorbeugung und Behandlung hormondefizienzbedingter neurodegenerativer Erkrankungen.

34. Verwendung einer Verbindung der Formeln nach Anspruch 2 bis 14 zur Herstellung eines Arzneimittels zur Vorbeugung und Behandlung der Alzheimerschen Krankheit sowie hormondefizienzbedingter Beeinträchtigung von Gedächtnis- und Lernfähigkeit.

35. Verwendung einer Verbindung der Formeln nach Anspruch 2 bis 14 zur Herstellung eines Arzneimittels zur Vorbeugung und Behandlung der benignen Prostatahyperplasie (BPH).

36. Phärmazeutische Zusammensetzungen, enthaltend mindestens eine Verbindung gemäß Anspruch 1 sowie einen pharmazeutisch verträglichen Träger.
